# EUROPEAN PATENT SPECIFICATION

(11) **EP 4 175 936 B1**
(45) Date of publication and mention of the grant of the patent: **20.11.2024**
(21) Application number: 21735982.7
(22) Date of filing: 30.06.2021
(51) Int. Cl.: C07C 41/42, C07C 43/13

(54) **METHOXYPROPANOLS SEPARATION INCLUDING AZEOTROPIC DISTILLATION**
ABTRENNUNG VON METHOXYPROPANOLEN EINSCHLIESSLICH AZEOTROPER DESTILLATION
SÉPARATION DE MÉTHOXYPROPANOLS COMPRENANT UNE DISTILLATION AZÉOTROPIQUE

(30) Priority: 01.07.2020 EP 20183426; 29.10.2020 EP 20204566
(43) Date of publication of application: 10.05.2023
(73) Proprietor: BASF SE, 67056 Ludwigshafen am Rhein (DE); Dow Global Technologies LLC, Midland, MI 48674 (US)
(72) Inventor: TELES, Joaquim Henrique, 67056 Ludwigshafen am Rhein (DE); SAN PIO BORDEJE, Maria Angel, 67056 Ludwigshafen am Rhein (DE); SEGERS, Dylan, 2040 Antwerpen (BE); RIEDEL, Dominic, 67056 Ludwigshafen am Rhein (DE); KAPPERT, Emiel Jan, 67056 Ludwigshafen am Rhein (DE); WEIDENBACH, Meinolf, 21683 Stade (DE); VAN NEER, Franciscus Johannes Robertus, 4542 NM Hoek (NL)
(74) Representative: Altmann Stößel Dick Patentanwälte PartG mbB
(86) International application number: PCT/EP2021/068018
(87) International publication number: WO 2022/003032

(56) References cited:
- EP-A1- 0 425 893
- EP-A1- 1 375 462

## Description

The present invention relates to a process for separating 1-methoxypropan-2-ol in a stream S3 from an aqueous stream comprising 1-methoxypropan-2-ol and 2-methoxypropan-1-ol, wherein the process comprises providing a stream S0 comprising 1-methoxypropan-2-ol, 2-methoxypro-pan-1-ol and water, and having a molar ratio of 1-methoxypropan-2-ol : 2-methoxypropan-1-ol in the range of from 1 : 5 to 5 : 1.

Propylene oxide (PO) is one of the most important chemical intermediates in industry. It represents the starting compound for a broad spectrum of products, such as foams, solvents or deicing agents. Traditionally, PO is produced via the chlorohydrin process, which is still in use today, as well as the oxirane method. The development of catalysts based on zeolitic materials having a framework structure comprising Si, O, and Ti, such as titanium silicalite-1, together with the improved availability of large quantities of hydrogen peroxide enabled the large-scale implementation of the co-product-free HPPO technology. This new process enables PO to be produced with excellent yields and selectivities.

The HPPO process produces propylene oxide from propylene and hydrogen peroxide in aqueous organic solvents with zeolitic materials having a framework structure comprising Si, O, and Ti as catalysts, in one constellation, methanol is used as the solvent, typically in combination with a zeolitic material having a framework structure comprising Si, O, and Ti of framework type MFI (titanium silicalite-1, TS-1) as catalyst. As side products in the HPPO process, methoxypropanols (MOPs) are formed by reaction of methanol (MeOH) and PO. MOPs are formed as a mixture of the isomers 1-methoxypropan-2-ol and 2-methoxypropan-1-ol. Thereof, 1-methoxy-propan-2-ol is an interesting compound, which can be used, for example, as a solvent. 2-Meth-oxypropan-1-ol on the other hand is teratogenic and has to be removed.

In an HPPO plant, MeOH, after having being used as solvent in the formation of PO, is normally separated from undesired side components and recovered. The MeOH containing stream, as mentioned above, comprises several side products such as the 1-methoxypropan-2-ol, which are of interest for further use. However, due to the similarities between the side products, the isolation of pure 1-methoxypropan-2-ol is complicated. First, the separation of both isomers is a difficult task, especially when considering the low concentration of water that is required for the final product (official sales specification is 100 ppm). Second, it is quite complicated to get rid of other side products, for example, propylene glycol dimethyl ether.

US 5,723,024 A describes a method for recovering 2-methyl-1-propanol from a mixture consisting of 2-methyl-1-propanol and 1-butanol. EP 0 425 893 A discloses a separation method for the two MOP isomers. DE 10233388 A1 describes MOP separation in general, but not specifically the separation of both MOP isomers. US 2004/0000473 A1 refers to the separation of the two MOP isomers from aqueous solution by usage of dewatering agents, without separation of both isomers. CN 103342631 and CN 103992214 are both related to MOP separation with extracting agents.

EP 1 375 462 A1 refers to a process of separating 1-methoxy-2-propanol and 2-methoxy-1-propanol from aqueous compositions, comprising dewatering of the aqueous composition comprising 1-methoxy- 2-propanol and 2-methoxy-1-propanol to a concentration of 1-methoxy-2-propanol and 2-methoxy-1-propanol of at least 90 percent by weight in total and isolation of 1-methoxy-2-propanol, 2-methoxy-1-propanol or mixtures thereof by means of distillation. The dewatering can be done by azeotropic distillation using an additive or by extractive distillation. The aqueous composition can be concentrated prior to the dewatering step by means of a pre-distillation to contain at least 10 percent by weight in total of 1-methoxy-2-propanol and 2-methoxy-1-propanol. The distillate obtained by such a distillation preferably contains the MOP isomers in an amount of 10 to 50 percent by weight. According to EP 1 375 462 A1, any suitable distillation column can be used for the pre-distillation step. The distillation column used for the pre-distillation can, according to EP 1 375 462 A1, be run at any suitable pressure; however, a pressure in the range of 0.5 to 5 bar is indicated as preferred embodiment, whereas atmospheric pressure is indicated as most preferred.

In view of the plurality of side products which can be present in a stream comprising the two MOP isomers, there is still a need to separate the 1-methoxypropan-2-ol in high purity. Especially, it had been found that removal of specific impurities, such as propylene glycol dimethyl ether, cannot, or at least not efficiently, be achieved by the methods known in the art.

It was therefore an object of the present invention to provide a process for 1-methoxypropan-2-ol separation, which is effective and allows to achieve 1-methoxypropan-2-ol in high purity with only traces of water (official sales specification is 100 ppm), as well as only traces of impurities.

In a first aspect, the invention thus relates to a process for separating 1-methoxypropan-2-ol from an aqueous stream comprising 1-methoxypropan-2-ol and 2-methoxypropan-1-ol, wherein the process comprises:
(a) Providing a stream S0 comprising 1-methoxypropan-2-ol, 2-methoxypropan-1-ol and water, and having a molar ratio of 1-methoxypropan-2-ol : 2-methoxypropan-1-ol in the range of from 1 : 5 to 5 : 1;
(b) separating 1-methoxypropan-2-ol and 2-methoxypropan-1-ol from the stream S0 provided in (a) by distillation comprising subjecting the stream S0 provided in (a) to distillation conditions in a distillation unit comprising a distillation column B, obtaining a (top) stream S1 comprising 1-methoxypropan-2-ol, 2-methoxypropan-1-ol and water, which is enriched in 1-methoxypropan-2-ol and 2-methoxypropan-1-ol compared to the stream S0 and a bottoms stream S1a comprising water and being depleted of 1-methoxypropan-2-ol and 2-methoxypropan-1-ol compared to S0; wherein the distillation column B is operated at a pressure of ≥ 2 bar;
(c) azeotropic distillation of the stream S1 obtained in (b) with an entrainer, comprising subjecting the stream S1 to azeotropic distillation conditions in an azeotropic distillation unit comprising a distillation column C, obtaining a bottoms stream S2 which is depleted of water and further enriched in 1-methoxypropan-2-ol and 2-methoxypropan-1-ol compared to the stream S1 and a top stream S2a comprising water and the entrainer;
(d) separating 1-methoxypropan-2-ol from the stream S2 obtained in (c) by distillation, comprising subjecting the stream S2 obtained in (c) to distillation conditions in a distillation unit comprising a distillation column D, obtaining a stream S3 comprising ≥ 95 weight-% 1-methoxypropan-2-ol and ≤ 0.5 weight-% of 2-methoxypropan-1-ol, based on the total weight of stream S3, and a stream S4 comprising ≥ 95 weight-% 2-methoxypropan-1-ol based on the total weight of stream S4;
(e) optionally distillative separation of the stream S2a comprising water and the entrainer obtained in (c) in a steam stripping column E using steam as stripping agent, obtaining a stream S5 comprising the agent, which is depleted of water compared to the stream S2a, and an aqueous stream S6 being depleted of the entrainer compared to S2a; and optionally recirculating at least a part of the stream S5 to (c).

Preferably, stream S0 comprises ≥ 0.001 weight-%, more preferred ≥ 0.006 weight-%, propylene glycol dimethyl ether (1,2-dimethoxypropane) based on the total weight of S0; and stream S3 preferably comprises less than 0.01 weight-%, more preferred less than 0.006 weight-%, of propylene glycol dimethyl ether based on the total weight of S3.

Surprisingly, it was found that if the distillation column B is operated at a pressure of ≥ 2 bar, it is possible to separate the propylene glycol dimethyl ether very efficiently, without need for further separation stages. It was found that operating column B at 1 bar (about atmospheric pressure) resulted in more water going over the top of column B with stream S1, which included the isomers 1-methoxypropan-2-ol and 2-methoxypropan-1-ol to the column C. Further, more impurities were transferred together with the water/1-methoxylpronan-2-ol, 2-methoxypropan-1-ol azeotrope to column C; mostly propylene glycol dimethyl ether (more than 89% more than at 10 bar). Consequently, also the impurity content in the final stream S3 was higher when column B was operated at 1 bar, resulting in a slightly lower purity of the obtained 1-methoxypropanol-2, and, simultaneously, in a propylene glycol dimethyl ether content in S3 of more than 0.006 weight-%, indeed more than 0.01 weight-%, based on the total weight of S3.

Preferably, (a), (b), (c) and (d) and optionally also (e) are operated in batch mode or in continuous mode, more preferred a (a), (b), (c) and (d) and optionally also (e) are operated in continuous mode.

Another aspect of the present invention is a process, which can be preferably conducted in a continuous mode and which is energy efficient. Thus, in a preferred embodiment of the process for separating 1-methoxypropan-2-ol from an aqueous stream comprising 1-methoxypropan-2-ol and 2-methoxypropan-1-ol, the thermal energy of stream S1 is partly transferred to a heat transfer medium stream HTMS1 after (b) and before step (c), preferably in a heat exchanger H, obtaining a heat transfer medium stream HTMS1a having an increased thermal energy content compared to HTMS1;
wherein HTMS1a is used to provide thermal energy to:
- the azeotropic distillation unit of step (c), preferably to a heat exchanger unit connected to the distillation column of the azeotropic distillation unit of (c),
   and/or
- the distillation unit of (d), preferably to a heat exchanger unit connected to the distillation column of the distillation unit of (d).

According to a further preferred embodiment of the process for separating 1-methoxypropan-2-ol from an aqueous stream comprising 1-methoxypropan-2-ol and 2-methoxypropan-1-ol, HTMS1a is used to provide thermal energy to:
- the azeotropic distillation unit of step (c), preferably to a heat exchanger unit connected to the distillation column of the azeotropic distillation unit of (c),
   and
- the distillation unit of (d), preferably to a heat exchanger unit connected to the distillation column of the distillation unit of (d).

According to a further preferred embodiment of the process for separating 1-methoxypropan-2-ol from an aqueous stream comprising 1-methoxypropan-2-ol and 2-methoxypropan-1-ol, the thermal energy of the bottoms stream S1a of (b) is partly transferred to a heat transfer medium stream HTMS2, preferably in a heat exchanger H1, obtaining a heat transfer medium stream HTMS2a, which has an increased thermal energy content compared to HTMS2, wherein HTMS2a is used to provide thermal energy to the steam used as stripping agent in the steam stripping column of (e).

It was surprisingly found that if column B is operated at a pressure ≥ 2 bar, the involved units can be energetically coupled in a very efficient manner. At lower pressure, it is still possible to energetically couple the units, but not as efficiently, causing the energy consumption to increase drastically.

Preferably, the thermal energy provided by HTMS1a provides at least 90 % of the energy demand of the azeotropic distillation unit of (c), and/or of the distillation unit of (d), more preferred the thermal energy provided by HTMS1a provides at least 95 % of the energy demand of the azeotropic distillation unit of (c), and/or of the distillation unit of (d); more preferred the thermal energy provided by HTMS1a provides at least 98 %, more preferred 99 %, more preferred 100%, of the energy demand of the azeotropic distillation unit of (c), and/or of the distillation unit of (d). More preferred, the thermal energy provided by HTMS1a provides at least 90 % of the energy demand of the azeotropic distillation unit of (c), and of the distillation unit of (d), more preferred the thermal energy provided by HTMS1a provides at least 95 % of the energy demand of the azeotropic distillation unit of (c), and of the distillation unit of (d); more preferred the thermal energy provided by HTMS1a provides at least 98 %, more preferred at least 99 %, more preferred 100 %, of the energy demand of the azeotropic distillation unit of (c), and of the distillation unit of (d).

In a preferred embodiment of the process for separating 1-methoxypropan-2-ol from an aqueous stream comprising 1-methoxypropan-2-ol and 2-methoxypropan-1-ol, in the range of from 40 to 95% of the thermal energy of stream S1 is transferred to the heat transfer medium stream HTMS1 obtaining a heat transfer medium stream HTMS1a having an increased thermal energy content compared to HTMS1. Preferably, in the range of from 50 to 90 %, more preferred in the range of from 55 to 85 %, more preferred in the range of from 60 to 80 %, of the thermal energy of stream S1 is transferred to the heat transfer medium stream HTMS1. A transfer of thermal energy of, for example, 70 % means that, for example, stream S1 has a temperature of 177 °C before thermal energy transfer and of 120 °C after thermal energy transfer. This corresponds to an energy content of 9.18 MW, wherein 7.38 MW are transferred via HTMS1/HTHMS1a to the azeotropic distillation unit of (c), and 1.245 MW to the distillation unit of (d).

In a further preferred embodiment of the process for separating 1-methoxypropan-2-ol from an aqueous stream comprising 1-methoxypropan-2-ol and 2-methoxypropan-1-ol, the thermal energy provided by HTMS2a provides at least 90 % of the energy demand required for producing the steam used as stripping agent in the steam stripping column of (e), more preferred the thermal energy provided by HTMS2a provides at least 95 % of the energy demand required for producing the steam used as stripping agent in the steam stripping column of (e); more preferred the thermal energy provided by HTMS2a provides at least 98 %, more preferred at least 99 %, more preferred 100 %, of the energy demand required for producing the steam used as stripping agent in the steam stripping column of (e).

The heat transfer medium used for HTMS1/HTMS1a and for HTMS2/HTMS2a is preferably steam (H₂O_{gaseous}).

### Stream S0

According to step (a), a stream S0 is provided, which comprises 1-methoxypropan-2-ol, 2-methoxypropan-1-ol and water, and which has a molar ratio of 1-methoxypropan-2-ol : 2-methoxy-propan-1-ol in the range of from 1 : 5 to 5 : 1.

According to a preferred embodiment, stream S0 is a stream obtained from a HPPO process, more preferred from an HPPO process, wherein propylene oxide is prepared from propylene and hydrogen peroxide in aqueous methanol as solvent, preferably using a TS-1 catalyst, more preferred using a TS-1 fixed bed catalyst in a reaction zone. MOPs are formed as side products in the process by reaction of MeOH and PO and are removed from the reaction zone with an initial MeOH containing product stream. From said initial product stream, MeOH is recovered, optionally using one or more further separation steps. According to one embodiment, an intermediate stream comprising MeOH, water and other components including the two MOP isomers is obtained. MeOH is then separated from water and the other components via distillation, wherein stream S0 is obtained, which comprises the two MOP isomers, water, and several other side components such as propylene glycol dimethyl ether (1,2-dimethoxypropane).

In a preferred embodiment of the process for separating 1-methoxypropan-2-ol from an aqueous stream comprising 1-methoxypropan-2-ol and 2-methoxypropan-1-ol, stream S0 provided in (a) comprises water in an amount in the range of from 50 to 90 weight-%, more preferred in the range of from 55 to 85 weight-% and a mixture of 1-methoxypropan-2-ol and 2-methoxypropan-1-ol in an amount in the range of from 8 to 50 weight-%, preferably in the range of from 13 to 45 weight-%, each based on the total weight of stream S0, the remaining amount up to 100 weight-% being other components (impurities and solvent (MeOH)).

Preferably, stream S0 provided in (a) comprises 1-methoxypropan-2-ol and 2-methoxypropan-1-ol in a molar ratio in the range of from 1 : 4 to 4 : 1; more preferred in the range of from 1 : 3 to 3 : 1, more preferred in the range of from 1 : 2 to 2 : 1, more preferred in the range of from 0.75 : 1 to 1 : 0.75. Preferably, stream S0 provided in (a) comprises propylene glycol dimethyl ether in an amount of ≥ 0.001 weight-%, more preferred ≥ 0.006 weight-%, more preferred in the range of from 0.001 to 0.1 weight-%, more preferred in the range of from 0.003 to 0.01 weight-%, more preferred in the range of from 0.004 to 0.01 weight-%, more preferred in the range of from 0.005 to 0.008 weight-%, based on the total weight of S0.

Propylene glycol dimethyl ether (1,2-dimethoxypropane) is an impurity contained in S0. Preferably, stream S0 comprises besides propylene glycol dimethyl ether in the range of from 0.0001 to 3 weight-%, more preferred in the range of from 0.001 to 2.5 weight-%, more preferred in the range of from 0.01 to 1.5 weight-%, each based on the total weight of S0, of one or more components selected from the group consisting of 1,1-dimethoxypropane, 1,2-propanediol (MPG), 1-butanol, 2,4-dimethyl-1,3-dioxolane, 2,6-dimethyl-4-heptanol, 2-butenal,2-ethyl-4-methyl-1,3-dioxolane, 2-hexanone, 2-methylcyclohexanol, 2-methylpentanal, 2-propen-1-ol, 4-methyl-1,3- dioxolane, acetaldehyde, 2-propanone, dimethoxymethane, dipropylene glycol, ethanol, hydroxyacetone, 2-propanol, methanol, acetic acid methyl ester, formic acid methyl ester, propylene oxide, tripropylene glycol, and dipropylene glycol monomethyl ether (DPGME). These components are here understood as solvent (MeOH) and further impurites.

### Column B

According to step(b), 1-methoxypropan-2-ol and 2-methoxypropan-1-ol are separated from the stream S0 provided in (a) by distillation comprising subjecting the stream S0 provided in (a) to distillation conditions in a distillation unit comprising a distillation column B. Preferably, the distillation column B comprised in the distillation unit according to (b) has between 5 and 100 theoretical stages, more preferred between 8 and 60 theoretical stages, more preferred between 10 and 40 theoretical stages, more preferred between 15 and 30 theoretical stages.

According to step (b), column B is operated at a pressure f ≥ 2 bar. Preferably, the distillation column B comprised in the distillation unit according to (b) is operated at a pressure in the range of from 2 to 30 bar, more preferred in the range of from > 3.5 to 20 bar, more preferred in the range of from 4 to 20 bar, more preferred in the range of from >5 to 15 bar, more preferred in the range of from 5.5 to 15 bar, more preferred in the range of from 6 to 14 bar, more preferred in the range of from 7 to 12 bar.

Regarding specific temperatures to be used at the top or at the bottom of distillation column B, no specific restrictions exists as long as a stream S1 and a stream S1a according to (b) are obtained. Preferably, the distillation column B comprised in the distillation unit according to (b) is operated at a temperature at the top of the distillation column B in the range of from 140 to 250 °C, more preferred in the range of from 150 to 220 °C, more preferred in the range of from 160 to 200 °C, more preferred in the range of from 170 to 190 °C. Preferably, the distillation column B comprised in the distillation unit according to (b) is operated at a temperature at the bottom in the range of from 140 to 250 °C, more preferred in the range of from 150 to 220 °C, more preferred in the range of from 160 to 200 °C, more preferred in the range of from 170 to 190 °C.

Regarding specific reflux conditions to be used in the distillation column B, no specific restrictions exists as long as a stream S1 and a stream S1a according to (b) are obtained. Preferably, the distillation column B comprised in the distillation unit according to (b) is operated with a reflux ratio in the range of from 1 to 10 g/g, more preferred in the range of from 2 to 9 g/g, more preferred in the range of from 3 to 8 g/g, more preferred in the range of from 4 to 6 g/g.

According to a preferred embodiment of the process for separating 1-methoxypropan-2-ol from an aqueous stream comprising 1-methoxypropan-2-ol and 2-methoxypropan-1-ol, the distillation column B comprised in the distillation unit according to (b) has an energy demand in the range of from 10 to 30 MW; wherein preferably the reboiler of distillation column B has an energy demand in the range of from 5 to 15 MW and the condenser of distillation column B has an energy demand in the range of from 5 to 15 MW.

### Stream S1

In step (b), a (top) stream S1 comprising 1-methoxypropan-2-ol, 2-methoxypropan-1-ol and water, which is enriched in 1-methoxypropan-2-ol and 2-methoxypropan-1-ol compared to the stream S0 is obtained. Preferably, ≥ 95 weight-% of stream S1, which leaves distillation column B over the top, consist of water,1-methoxypropan-2-ol and 2-methoxypropan-1-ol. More preferred stream S1 comprises water in a amount in the range of from 40 to 80 weight-%, more preferred in the range of from 50 to 70 weight-%, more preferred in the range of from 55 to 65 weight-%; and a mixture of 1-methoxypropan-2-ol and 2-methoxypropan-1-ol in an amount in the range of from 20 to 60 weight-%, preferably in the range of from 30 to 50 weight-%, more preferred in the range of from 35 to 45 weight-%, each based on the total weight of stream S1, wherein preferably stream S1 comprises 1-methoxypropan-2-ol and 2-methoxypropan-1-ol in a molar ratio in the range of from 1 : 4 to 4 : 1; preferably in the range of from 1 : 3 to 3 : 1, more preferred in the range of from 1 : 2 to 2 : 1, more preferred in the range of from 0.75 : 1 to 1 : 0.75.

Preferably, stream S1, which leaves distillation column B over the top, contains less than 0.05 weight-%, more preferred less than 0.005 weight-%, more preferred less than 0.004 weight-%, more preferred less than 0.002 weight-%, of propylene glycol dimethyl ether, based on the total weight of S1. The wording "less than" means from 0 to less than the respective value.

According to some embodiments of the process for separating 1-methoxypropan-2-ol from an aqueous stream comprising 1-methoxypropan-2-ol and 2-methoxypropan-1-ol, stream S1 after leaving column B and before entering column C is flashed to reduce the heat demand in column C.

### Stream S1a

In step (b), also a bottoms stream S1a is obtained, which comprises water and is depleted of 1-methoxypropan-2-ol and 2-methoxypropan-1-ol compared to S0. Preferably, stream S1a comprises ≥ 95 weight-% of water. More preferred less than 1 weight-% of stream S1a consist of 1-methoxypropan-2-ol and 2-methoxypropan-1-ol, each based on the total weight of stream S1a, more preferred S1a comprises ≥ 98 weight-% of water, and less than 1 weight-% of stream S1a consist of 1-methoxypropan-2-ol and 2-methoxypropan-1-ol; more preferred S1a comprises ≥ 99 weight-% of water, and less than 1 weight-% of stream S1a consist of 1-methoxypropan-2-ol and 2-methoxypropan-1-ol.

### Column C

According to step (c), the stream S1 obtained in (b) is azeotropically distilled with an entrainer, comprising subjecting the stream S1 to azeotropic distillation conditions in an azeotropic distillation unit comprising a distillation column C, obtaining a bottoms stream S2 which is depleted of water and further enriched in 1-methoxypropan-2-ol and 2-methoxypropan-1-ol compared to the stream S1 and a top stream S2a comprising water and the entrainer.

Preferably, distillation column C comprised in the azeotropic distillation unit according to (c) has between 2 and 50 theoretical stages, more preferred between 5 and 25 theoretical stages, more preferred between 10 and 20 theoretical stages. Preferably, the distillation column C comprised in the azeotropic distillation unit according to (c) is operated at a pressure in the range of from 0.5 to 10 bar, more preferred in the range of from 1 to 5 bar, more preferred in the range of from 1.5 to 2.5 bar. Preferably, the distillation column C comprised in the azeotropic distillation unit according to (c) is operated at a bottoms temperature in the range of from 120 to 180 °C, more preferred in the range of from 130 to 170 °C, more preferred in the range of from 140 to 160 °C. Preferably, the distillation column C comprised in the azeotropic distillation unit according to (c) is operated at a temperature at the top of the column in the range of from 70 to 110 °C, more preferred in the range of from 80 to 100 °C, more preferred in the range of from 85 to 95 °C. In some embodiments, the distillation column C comprised in the azeotropic distillation unit according to (c) is operated with a reflux ratio in the range of from 10 to 40 g/g, preferably in the range of from 12 to 30 g/g, more preferred in the range of from 15 to 25 g/g.

In a preferred embodiment of the process for separating 1-methoxypropan-2-ol from an aqueous stream comprising 1-methoxypropan-2-ol and 2-methoxypropan-1-ol, the distillation column C comprised in the azeotropic distillation unit according to (c) has an energy demand in the range of from 10 to 30 MW; wherein preferably the reboiler of distillation column C has an energy demand in the range of from 5 to 15 MW and the condenser of distillation column C has an energy demand the range of from 5 to 15 MW.

### Entrainer

In step (c), stream S1 obtained in (b) is azeotropically distillated with an entrainer. Generally, no restrictions exist in what kind of entrainer is used as long as the streams S2 and S2a are obtained. Preferably, the entrainer is selected from the group consisting of aromatic hydrocarbons, cyclic hydrocarbons, acyclic hydrocarbons, esters of carbonic acids, ketones, and mixtures of two or more of these compounds; more preferred from the group consisting of toluene, benzene, hexane, cyclohexane, C₁-C₅ alkyl esters of carbonic acid, C₂-C₁₀ ketones and mixtures of two or more of these compounds; more preferred from the group consisting of benzene, cyclohexane, dimethyl carbonate, 2-pentanon and mixtures of two or more of these compounds; more preferred from the group consisting of benzene, cyclohexane, 2-pentanon and mixtures of two or more of these compounds; more preferred the entrainer at least comprises benzene.

The azeotropic distillation with an entrainer in column C is necessary in order to break the azeotrope and separate the water from the 1-methoxy-2-propanol and 2-methoxy-1-propanol. In the azeotrope, the boiling points of 1-methoxy-2-propanol and 2-methoxy-1-propanol/water are so close to each other that there is no separation possible of 1-methoxypropanol from 2-methoxypropanol. When the water is removed (in this case via azeotropic distillation), the boiling points of the two methoxypropanols are different enough from each other, enabling their separation.

The entrainer is applied to column C in a weight-based ratio stream S1 entering column C : entrainer entering column C in the range of from 1 : 5 to 5 : 1, preferably in the range of from 1 : 0.5 to 1 : 7, more preferred in the range of from 1 : 0.8 to 1 : 6.5, more preferred in the range of from 1 : 1 to 1 : 6. The entrainer stream entering column C consists of one or more streams and comprises preferably fresh entrainer and/or recycled entrainer, more preferred fresh entrainer and recycled entrainer. Preferably, in the range of from 95 to 100 weight-% of the entrainer stream entering column C consists of fresh entrainer and recycled entrainer. Preferably, during operation, fresh entrainer is added to column C in a weight based ratio stream S1 entering column C : fresh entrainer entering column C in the range of from 100 : 1 to 1500 : 1, preferably in the range of from 200 : 1 to 1200 : 1, more preferred in the range of from 300 : 1 to 1100 : 1. Preferably, during operation, recycled entrainer is added to column C in a weight based ratio stream S1 entering column C : recycled entrainer entering column C 1 : 5 to 5 : 1, preferably in the range of from 1 : 0.5 to 1 : 5.5, more preferred in the range of from 1 : 1.5 to 1 : 5. Regarding details about recycled entrainer, reference is made to the section referring to stream S5 below.

### Stream S2

In step (c), a stream S2 is obtained from the azeotropic distillation, which is depleted of water and further enriched in 1-methoxypropan-2-ol and 2-methoxypropan-1-ol compared to the stream S1. Preferably, the stream S2 is obtained as bottoms stream from column C, wherein ≥ 85 weight-%, more preferred ≥ 90 weight-%, more preferred ≥ 95 weight-%, more preferred ≥97 weight-%, ≥ 98 weight-%, more preferred ≥ 99 weight-% of S2 consisted of 1-methoxy-2-propanol and 2-methoxy-1-propanol, based on the total weight of S2, wherein preferably stream S2 comprises 1-methoxypropan-2-ol and 2-methoxypropan-1-ol in a molar ratio in the range of from 1 : 4 to 4 : 1; preferably in the range of from 1 : 3 to 3 : 1, more preferred in the range of from 1 : 2 to 2 : 1, more preferred in the range of from 0.75 : 1 to 1 : 0.75. Preferably, S2 has a water content of at the outmost 100 ppm of water, based on the total weight of S2. Preferably, S2 contains less than 100 ppm, more preferred less than 10 ppm, more preferred less than 1 ppm of entrainer, based on the total weight of S2.

### Stream S2a

In step (c), a further top stream S2a is obtained from the azeotropic distillation, which comprises water and the entrainer. Preferably, stream S2a is obtained as tom stream from distillation column C, wherein 90 weight-%, more preferred ≥ 95 weight-%, more preferred ≥97 weight-%, ≥ 98 weight-%, more preferred ≥ 99 weight-% of S2a consisted of water and the entrainer based on the total weight of S2a; wherein preferably the molar ratio of water : entrainer in the stream S2a is in the range of from 1 : 4 to 1 : 12, preferably in the range of from 1 : 6 to 1 : 10, more preferred in the range of from 1 : 8 to 1 : 9.

### Column D

According to step (d), 1-methoxypropan-2-ol is separated from the stream S2 obtained in (c) by distillation, comprising subjecting the stream S2 obtained in (c) to distillation conditions in a distillation unit comprising a distillation column D. Preferably, the distillation column D comprised in the distillation unit according to (d) has between 20 and 100 theoretical stages, more preferred between 30 and 80 theoretical stages, more preferred between 40 and 60 theoretical stages, more preferred between 45 and 52 theoretical stages. Preferably, the distillation column D comprised in the distillation unit according to (d) is operated at a pressure in the range of from 0.5 to 10 bar, more preferred in the range of from 1 to 5 bar, more preferred in the range of from 2 to 4 bar. Preferably, the distillation column D comprised in the distillation unit according to (d) is operated at a bottoms temperature in the range of from 140 to 200 °C, more preferred in the range of from 150 to 190 °C, more preferred in the range of from 160 to 180 °C. Preferably, the distillation column D comprised in the distillation unit according to (d) is operated at a temperature at the top of the column in the range of from 120 to 200 °C, more preferred in the range of from 130 to 180 °C, more preferred in the range of from 140 to 165 °C. In some embodiments, the distillation column D comprised in the distillation unit according to (d) is operated with a reflux ratio in the range of from 5 to 30 g/g, preferably in the range of from 8 to 20 g/g, more preferred in the range of from 10 to 15 g/g.

Preferably, the distillation column D comprised in the distillation unit according to (d) has an energy demand in the range of from 1 to 5 MW; wherein preferably the reboiler of distillation column D has an energy demand in the range of from 1 to 1.5 MW and the condenser of distillation column D has an energy demand the range of from 1 to 1.5 MW. For example, having 48 theoretical stages in distillation column D would result in an energy demand of 1.245 MW in the reboiler of distillation column D and 1.23 MW in the condenser of distillation column D. In case of decreasing the theoretical stages from 48 to 40, the heat duty would increase from 1.245 MW to 12 MW in the reboiler, i.e. the energy demand would increase by about factor 10.

### Stream S3

From step (d), a stream S3 is obtained, which comprises ≥ 95 weight-% 1-methoxypropan-2-ol and ≤ 0.5 weight-% of 2-methoxypropan-1-ol, based on the total weight of stream S3. In some embodiments, the stream S3 is removed as top stream from distillation column D, which comprises ≥ 95 weight-% 1-methoxypropan-2-ol and ≤ 0.5 weight-% of 2-methoxypropan-1-ol, preferably ≥ 98 weight-% 1-methoxypropan-2-ol and ≤ 0.15 weight-% of 2-methoxypropan-1-ol, more preferred ≥ 99 weight-% 1-methoxypropan-2-ol and ≤ 0.1 weight-% of 2-methoxypropan-1-ol, each based on the total weight of stream S3. Preferably, the stream S3 comprises preferably less than 0.01 weight-%, more preferred less than 0.008 weight-%, more preferred less than 0.007 weight-%, more preferred less than 0.006 weight-%, of propylene glycol dimethyl ether based on the total weight of stream S3.

According to a preferred embodiment, stream S3 comprises ≥ 95 weight-% 1-methoxypropan-2-ol and ≤ 0.5 weight-% of 2-methoxypropan-1-ol, preferably ≥ 98 weight-% 1-methoxypropan-2-ol and ≤ 0.15 weight-% of 2-methoxypropan-1-ol, more preferred ≥ 99 weight-% 1-methoxypro-pan-2-ol and ≤ 0.1 weight-% of 2-methoxypropan-1-ol, each based on the total weight of stream S3, and stream S3 comprises preferably less than 0.01 weight-%, more preferred less than 0.008 weight-%, more preferred less than 0.007 weight-%, more preferred less than 0.006 weight-%, of propylene glycol dimethyl ether based on the total weight of stream S3.

Stream S3 comprises the valuable product 1-methoxypropan-2-ol in a purity ≥ 95 weight-%, while the detrimental impurities 2-methoxypropan-1-ol and propylene glycol dimethyl ether are only present in amounts of ≤ 0.15 weight-% and < 0.01 weight-% respectively. Furthermore, stream S3 comprises less than 100 ppm, more preferred less than 50 ppm of water based on the total weight of stream S3, i.e. the final product 1-methoxypropan-2-ol fulfills the official sales specification of ≤ 100 ppm.

### Stream S4

From step (d), also a stream S4 is obtained, which comprises ≥ 95 weight-% 2-methoxypropan-1-ol based on the total weight of stream S4. Preferably, the stream S4 is removed as bottoms stream from distillation column D, wherein S4 comprises ≥ 95 weight-% 2-methoxypropan-1-ol and ≤ 0.5 weight-% 1-methoxypropan-2-ol, preferably ≥ 96 weight-% 2-methoxypropan-1-ol and ≤ 0.1 weight-% 1-methoxypropan-2-ol, more preferred ≥ 98 weight-% 2-methoxypropan-1-ol and ≤ 0.001 weight-% 1-methoxypropan-2-ol, more preferred ≥ 99 weight-% 2-methoxypropan-1-ol and ≤ 0.0001 weight-% 1-methoxypropan-2-ol, each based on the total weight of stream S4.

2-Methoxypropan-1-ol is a teratogenic component. Thus, its concentration in the valuable 1-methoxypropan-2-ol stream has to be severely restricted, preferably to a maximum of 0.3 weight-%, based on the total weight of the respective stream. Based on the process of the present invention, the following specifications (instreams S3 and S4) can be met: 0.05% 2-methoxypropan-1-ol in S3 and 300 ppm of 1-methoxypropan-2-ol in S4.

### Column E

In some embodiments of the process for separating 1-methoxypropan-2-ol from an aqueous stream comprising 1-methoxypropan-2-ol and 2-methoxypropan-1-ol, optionally a distillative separation of the stream S2a comprising water and the entrainer obtained in (c) is done in a steam stripping column E using steam as stripping agent. Preferably, the steam stripping column E according to (e) has between 3 and 20 theoretical stages, more preferred between 5 and 15 theoretical stages, more preferred between 8 and 12 theoretical stages.

Preferably, the steam stripping column E according to (e) is operated at a pressure in the range of from 0.1 to 5 bar, more preferred in the range of from 0.5 to 2 bar, more preferred in the range of from 0.8 to 1.2 bar. Preferably, the steam stripping column E according to (e) is operated at a temperature at the top of the distillation column B in the range of from 60 to 100 °C, more preferred in the range of from 70 to 90 °C, more preferred in the range of from 75 to 85 °C. Preferably, the steam stripping column E according to (e) is operated at a temperature at the bottom in the range of from 80 to 120 °C, more preferred in the range of from 85 to 115 °C, more preferred in the range of from 90 to 110 °C, more preferred in the range of from 95 to 105 °C.

Regarding a steam stripping agent, the steam stripping column E according to (e) is preferably operated with steam (H₂O_{gaseous}), which is preferably introduced into steam stripping column E in the range of from 80 to 120 kg/h, more preferred in the range of from 90 to 110 kg/h, more preferred in the range of from 95 to 105 kg/h; wherein the gaseous water has preferably a pressure in the range of from 2 to 6 bar, more preferred in the range of from 3 to 5 bar, more preferred in the range of from 3.5 to 4.5 bar. Preferably, the gaseous water has a temperature in the range of from 120 to 160 °C, more preferred in the range of from 130 to 150 °C.

### Stream S5

In optional step (e), a stream S5 comprising the entrainer is obtained, which is depleted of water compared to the stream S2a. At least a part of Stream S5 is optionally recirculating to step (c). The stream S5 is preferably removed from the steam stripping column E as top stream, wherein stream S5 preferably comprises ≥ 25 weight-%, preferably ≥ 30 weight-%, entrainer.

### Stream S6

Furthermore, a stream S6 being depleted of the entrainer compared to S2a is obtained in optional step (e). The stream S6 is preferably removed from the steam stripping column E as bottoms stream, wherein stream S6 comprises ≥ 95 weight-% water and ≤ 1 weight-% entrainer, preferably ≥ 96 weight-% water and ≤ 0.1 weight-% entrainer, more preferred ≥ 97 weight-% water and ≤ 0.01 weight-% entrainer, more preferred ≥ 99 weight-% water and ≤ 0.0001 weight-% entrainer.

### Possible decanter

In some embodiments of the process for separating 1-methoxypropan-2-ol from an aqueous stream comprising 1-methoxypropan-2-ol and 2-methoxypropan-1-ol, at least a part of stream S2a, after removal from distillation column C and prior to entering steam stripping column E, is introduced into a decanter, wherein at least a part pf the entrainer being contained in said part of stream S2a is separated from stream S2a obtaining a stream S2a-1 comprising entrainer and a stream S2a-2 being depleted of entrainer compared to S2a, wherein stream S2a-1 is preferably recycled to distillation column C and stream S2a-2 is introduced into steam stripping column E.

### Further column F

In some embodiments the process for separating 1-methoxypropan-2-ol from an aqueous stream comprising 1-methoxypropan-2-ol and 2-methoxypropan-1-ol, preferably if the methanol content in stream S0 is > 0.1 weight-%, more preferred in the range of from 0.02 to 1.00 weight-%, each based on the total weight of stream S0, further comprises the step:
f) separating methanol from stream S1 by distillation, comprising subjecting the stream S1 obtained in (b) to distillation conditions in a distillation unit comprising a distillation column F, obtaining a stream S1' comprising 1-methoxypropan-2-ol, 2-methoxypropan-1-ol and ≤ 0.1 weight-% methanol based on the total weight of S1', wherein S1' is introduced in step (c) into extractive column C instead of stream S1;
   or
g) separating methanol from stream S6 by distillation, comprising subjecting the stream S6 obtained in (d) to distillation conditions in a distillation unit comprising a distillation column F, obtaining a stream S6', which comprises ≥ 95 weight-% water, ≤ 1 weight-% entrainer and ≤ 0.1 weight-% methanol, preferably ≥ 96 weight-% water, ≤ 0.1 weight-% entrainer and ≤ 0.1 weight-% methanol;
wherein preferably step (g) is used.

Distillation column F preferably comprises in the range of from 5 to 25 theoretical stages, more preferred in the range of from 7 to 15 theoretical stages, more preferred in the range of from 8 to 12 theoretical stages; and/or wherein distillation column F is operated at a pressure in the range of from 0.5 to 1.5 bar, preferably in the range of from 0.8 to 1.2 bar; and/or wherein distillation column F has an energy demand in the range of from 1 to 3 MW; preferably an energy demand in the condenser in the range of from 0.5 to 1.5 MW and/or an energy demand in the range of from 0.5 to 1.5 MW in the reboiler.

### 1-Methoxypropan-2-ol

Even if not being subject-matter of the present invention, it is possible to obtain from the above-described process 1-methoxypropan-2-ol.

Furthermore, a mixture of 1-methoxypropan-2-ol and 2-methoxypropan-1-ol can be obtained from the above-described process, which preferably comprises in the range of from 95 to 100 weight-% 1-methoxypropan-2-ol and ≤ 0.5 weight-% of 2-methoxypropan-1-ol, more preferred in the range of from 98 to 100 weight-% 1-methoxypropan-2-ol and ≤ 0.15 weight-% of 2-methoxy-propan-1-ol, more preferred in the range of from 99 to 100 weight-% 1-methoxypropan-2-ol and ≤ 0.1 weight-% of 2-methoxypropan-1-ol, each based on the total weight of the mixture.

1-Methoxypropan-2-ol or a mixture of 1-methoxypropan-2-ol and 2-methoxypropan-1-ol can be used for the production of methoxypropyl acetate or methoxyisopropylamine.

The present invention is further illustrated by the following set of embodiments and combinations of embodiments resulting from the dependencies and back-references as indicated. In particular, it is noted that in each instance where a range of embodiments is mentioned, for example in the context of a term such as " any one of embodiments (1) to (4) ",, every embodiment in this range is meant to be explicitly disclosed for the skilled person, i.e. the wording of this term is to be understood by the skilled person as being synonymous to "any one of embodiments (1), (2), (3), and (4)". Further, it is explicitly noted that the following set of embodiments is not the set of claims determining the extent of protection, but represents a suitably structured part of the description directed to general and preferred aspects of the present invention.

According to an embodiment (1), the present invention relates to process for separating 1-methoxypropan-2-ol from an aqueous stream comprising 1-methoxypropan-2-ol and 2-methoxypropan-1-ol, wherein the process comprises:
(a) Providing a stream S0 comprising 1-methoxypropan-2-ol, 2-methoxypropan-1-ol and water, and having a molar ratio of 1-methoxypropan-2-ol : 2-methoxypropan-1-ol in the range of from 1 : 5 to 5 : 1;
(b) separating 1-methoxypropan-2-ol and 2-methoxypropan-1-ol from the stream S0 provided in (a) by distillation comprising subjecting a the stream S0 provided in (a) to distillation conditions in a distillation unit comprising a distillation column B, obtaining a (top) stream S1 comprising 1-methoxypropan-2-ol, 2-methoxypropan-1-ol and water, which is enriched in 1-methoxypropan-2-ol and 2-methoxypropan-1-ol compared to the stream S0 and a bottoms stream S1a comprising water and being depleted of 1-methoxypropan-2-ol and 2-methoxypropan-1-ol compared to S0; wherein the distillation column B is operated at a pressure of ≥ 2 bar;
(c) azeotropic distillation of the stream S1 obtained in (b) with an entrainer, comprising subjecting the stream S1 to azeotropic distillation conditions in an azeotropic distillation unit comprising a distillation column C, obtaining a bottoms stream S2 which is depleted of water and further enriched in 1-methoxypropan-2-ol and 2-methoxypropan-1-ol compared to the stream S1 and a top stream S2a comprising water and the entrainer;
(d) separating 1-methoxypropan-2-ol from the stream S2 obtained in (c) by distillation, comprising subjecting the stream S2 obtained in (c) to distillation conditions in a distillation unit comprising a distillation column D, obtaining a stream S3 comprising ≥ 95 weight-% 1-methoxypropan-2-ol and ≤ 0.5 weight-% of 2-methoxypropan-1-ol, based on the total weight of stream S3, and a stream S4 comprising ≥ 95 weight-% 2-methoxypropan-1-ol based on the total weight of stream S4;
(e) optionally distillative separation of the stream S2a comprising water and the entrainer obtained in (c) in a steam stripping column E using steam as stripping agent, obtaining a stream S5 comprising the entrainer, which is depleted of water compared to the stream S2a, and an aqueous stream S6 being depleted of the entrainer compared to S2a; and optionally recirculating at least a part of the stream S5 to (c);
wherein stream S0 preferably comprises ≥ 0.001 weight-%, more preferred ≥ 0.006 weight-% propylene glycol dimethyl ether (1,2-dimethoxypropane) based on the total weight of S0; and stream S3 preferably comprises less than 0.01 weight-%, more preferred less than 0.006 weight-% of propylene glycol dimethyl ether based on the total weight of S3.

A preferred embodiment (2) concretizing embodiment (1) relates to said process for separating 1-methoxypropan-2-ol from an aqueous stream comprising 1-methoxypropan-2-ol and 2-methoxypropan-1-ol according to embodiment 1, wherein the thermal energy of stream S1 is partly transferred to a heat transfer medium stream HTMS1 after (b) and before step (c), preferably in a heat exchanger H, obtaining a heat transfer medium stream HTMS1a having an increased thermal energy content compared to HTMS1;
wherein HTMS1a is used to provide thermal energy to:
- the azeotropic distillation unit of step (c), preferably to a heat exchanger unit connected to the distillation column of the azeotropic distillation unit of (c),
   and/or
- the distillation unit of (d), preferably to a heat exchanger unit connected to the distillation column of the distillation unit of (d);

A further preferred embodiment (3) concretizing embodiment (2) relates to said process for separating 1-methoxypropan-2-ol from an aqueous stream comprising 1-methoxypropan-2-ol and 2-methoxypropan-1-ol, wherein HTMS1a is used to provide thermal energy to:
- the azeotropic distillation unit of step (c), preferably to a heat exchanger unit connected to the distillation column of the azeotropic distillation unit of (c),
   and
- the distillation unit of (d), preferably to a heat exchanger unit connected to the distillation column of the distillation unit of (d).

A further preferred embodiment (4) concretizing embodiment (2) or (3) relates to said process for separating 1-methoxypropan-2-ol from an aqueous stream comprising 1-methoxypropan-2-ol and 2-methoxypropan-1-ol, wherein the thermal energy of the bottoms stream S1a of (b) is partly transferred to a heat transfer medium stream HTMS2, preferably in a heat exchanger H1, obtaining a heat transfer medium stream HTMS2a, which has an increased thermal energy content compared to HTMS2, wherein HTMS2a is used to provide thermal energy to the steam used as stripping agent in the steam stripping column of (e).

A further preferred embodiment (5) concretizing any one of embodiments (2) to (4) relates to said process for separating 1-methoxypropan-2-ol from an aqueous stream comprising 1-methoxypropan-2-ol and 2-methoxypropan-1-ol, wherein the thermal energy provided by HTMS1a provides at least 90 % of the energy demand of the azeotropic distillation unit of (c), and/or of the distillation unit of (d), preferably the thermal energy provided by HTMS1a provides at least 95 % of the energy demand of the azeotropic distillation unit of (c), and/or of the distillation unit of (d); more preferred the thermal energy provided by HTMS1a provides at least 98 %, more preferred at least 99 %, more preferred 100%, of the energy demand of the azeotropic distillation unit of (c), and/or of the distillation unit of (d).

A further preferred embodiment (6) concretizing any one of embodiments (2) to (5) relates to process for separating 1-methoxypropan-2-ol from an aqueous stream comprising 1-methoxy-propan-2-ol and 2-methoxypropan-1-ol, wherein the thermal energy provided by HTMS1a provides at least 90 % of the energy demand of the azeotropic distillation unit of (c), and of the distillation unit of (d), preferably the thermal energy provided by HTMS1a provides at least 95 % of the energy demand of the azeotropic distillation unit of (c), and of the distillation unit of (d); more preferred the thermal energy provided by HTMS1a provides at least 98 %, more preferred at least 99 %, more preferred 100 %, of the energy demand of the azeotropic distillation unit of (c), and of the distillation unit of (d).

A further preferred embodiment (7) concretizing any one of embodiments (2) to (6) relates to process for separating 1-methoxypropan-2-ol from an aqueous stream comprising 1-methoxypropan-2-ol and 2-methoxypropan-1-ol, wherein in the range of from 40 to 95% of the thermal energy of stream S1 is transferred to the heat transfer medium stream HTMS1 obtaining a heat transfer medium stream HTMS1a having an increased thermal energy content compared to HTMS1.

A further preferred embodiment (8) concretizing any one of embodiments (2) to (7) relates to process for separating 1-methoxypropan-2-ol from an aqueous stream comprising 1-methoxypropan-2-ol and 2-methoxypropan-1-ol, wherein the thermal energy provided by HTMS2a provides at least 90 % of the energy demand required for producing the steam used as stripping agent in the steam stripping column of (e), preferably the thermal energy provided by HTMS2a provides at least 95 % of the energy demand required for producing the steam used as stripping agent in the steam stripping column of (e); more preferred the thermal energy provided by HTMS2a provides at least 98 %, more preferred at least 99 %, more preferred 100 %, of the energy demand required for producing the steam used as stripping agent in the steam stripping column of (e).

A further preferred embodiment (9) concretizing any one of embodiments (1) to (8) relates to process for separating 1-methoxypropan-2-ol from an aqueous stream comprising 1-methoxypropan-2-ol and 2-methoxypropan-1-ol, wherein stream S0 provided in (a) comprises water in an amount in the range of from 50 to 90 weight-%, preferably in the range of from 55 to 85 weight-% and a mixture of 1-methoxypropan-2-ol and 2-methoxypropan-1-ol in an amount in the range of from 8 to 50 weight-%, preferably in the range of from 13 to 45 weight-%, each based on the total weight of stream S0, the remaining amount up to 100 weight-% being other components (impurities and solvent (MeOH)).

A further preferred embodiment (10) concretizing any one of embodiments (1) to (9) relates to said process for separating 1-methoxypropan-2-ol from an aqueous stream comprising 1-methoxypropan-2-ol and 2-methoxypropan-1-ol, wherein stream S0 provided in (a) comprises 1-methoxypropan-2-ol and 2-methoxypropan-1-ol in a molar ratio in the range of from 1 : 4 to 4 : 1; preferably in the range of from 1 : 3 to 3 : 1, more preferred in the range of from 1 : 2 to 2 : 1, more preferred in the range of from 0.75 : 1 to 1 : 0.75.

A further preferred embodiment (11) concretizing any one of embodiments (1) to (10) relates to said process for separating 1-methoxypropan-2-ol from an aqueous stream comprising 1-methoxypropan-2-ol and 2-methoxypropan-1-ol, wherein stream S0 provided in (a) comprises propylene glycol dimethyl ether in an amount of ≥ 0.001 weight-%, more preferred ≥ 0.006 weight-%, more preferred in the range of from 0.001 to 0.1 weight-%, more preferred in the range of from 0.003 to 0.01 weight-%, more preferred in the range of from 0.004 to 0.01 weight-%, more preferred in the range of from 0.005 to 0.008 weight-%, based on the total weight of S0.

A further preferred embodiment (12) concretizing any one of embodiments (1) to (11) relates to said process for separating 1-methoxypropan-2-ol from an aqueous stream comprising 1-methoxypropan-2-ol and 2-methoxypropan-1-ol, wherein the distillation column B comprised in the distillation unit according to (b) has between 5 and 100 theoretical stages, preferably between 8 and 60 theoretical stages, more preferred between 10 and 40 theoretical stages, more preferred between 15 and 30 theoretical stages.

A further preferred embodiment (13) concretizing any one of embodiments (1) to (12) relates to said process for separating 1-methoxypropan-2-ol from an aqueous stream comprising 1-methoxypropan-2-ol and 2-methoxypropan-1-ol, wherein the distillation column B comprised in the distillation unit according to (b) is operated at a pressure in the range of from 2 to 30 bar, preferably in the range of from 3.5 to 20 bar, more preferred in the range of from 4 to 20 bar, more preferred in the range of from >5 to 15 bar, more preferred in the range of from 5.5 to 15 bar, more preferred in the range of from 6 to 14 bar, more preferred in the range of from 7 to 12 bar.

A further preferred embodiment (14) concretizing any one of embodiments (1) to (13) relates to said process for separating 1-methoxypropan-2-ol from an aqueous stream comprising 1-methoxypropan-2-ol and 2-methoxypropan-1-ol, wherein the distillation column B comprised in the distillation unit according to (b) is operated at a temperature at the top of the distillation column B in the range of from 140 to 250 °C, preferably in the range of from 150 to 220 °C, more preferred in the range of from 160 to 200 °C, more preferred in the range of from 170 to 190 °C.

A further preferred embodiment (15) concretizing any one of embodiments (1) to (14) relates to said process for separating 1-methoxypropan-2-ol from an aqueous stream comprising 1-methoxypropan-2-ol and 2-methoxypropan-1-ol, wherein the distillation column B comprised in the distillation unit according to (b) is operated at a temperature at the bottom in the range of from 140 to 250 °C, preferably in the range of from 150 to 220 °C, more preferred in the range of from 160 to 200 °C, more preferred in the range of from 170 to 190 °C.

A further preferred embodiment (16) concretizing any one of embodiments (1) to (15) relates to said process for separating 1-methoxypropan-2-ol from an aqueous stream comprising 1-methoxypropan-2-ol and 2-methoxypropan-1-ol, wherein the distillation column B comprised in the distillation unit according to (b) is operated with a reflux ratio in the range of from 1 to 10 g/g, preferably in the range of from 2 to 9 g/g, more preferred in the range of from 3 to 8 g/g, more preferred in the range of from 4 to 6 g/g.

A further preferred embodiment (17) concretizing any one of embodiments (1) to (16) relates to said process for separating 1-methoxypropan-2-ol from an aqueous stream comprising 1-methoxypropan-2-ol and 2-methoxypropan-1-ol, wherein the distillation column B comprised in the distillation unit according to (b) has an energy demand in the range of from 10 to 30 MW; wherein preferably the reboiler of distillation column B has an energy demand in the range of from 5 to 15 MW and the condenser of distillation column B has an energy demand the range of from 5 to 15 MW.

A further preferred embodiment (18) concretizing any one of embodiments (1) to (17) relates to said process for separating 1-methoxypropan-2-ol from an aqueous stream comprising 1-methoxypropan-2-ol and 2-methoxypropan-1-ol, wherein ≥ 95 weight-% of stream S1, which leaves distillation column B over the top, consist of water,1-methoxypropan-2-ol and 2-methoxypropan-1 -ol; wherein preferably stream S1 comprises water in a amount in the range of from 40 to 80 weight-%, preferably in the range of from 50 to 70 weight-%, more preferred in the range of from 55 to 65 weight-%; and a mixture of 1-methoxypropan-2-ol and 2-methoxypropan-1-ol in an amount in the range of from 20 to 60 weight-%, preferably in the range of from 30 to 50 weight-%, more preferred in the range of from 35 to 45 weight-%, each based on the total weight of stream S1, wherein preferably stream S1 comprises 1-methoxypropan-2-ol and 2-methoxypropan-1-ol in a molar ratio in the range of from 1 : 4 to 4 : 1; preferably in the range of from 1 : 3 to 3 : 1, more preferred in the range of from 1 : 2 to 2 : 1, more preferred in the range of from 0.75 : 1 to 1 : 0.75.

A further preferred embodiment (19) concretizing any one of embodiments (1) to (18) relates to said process for separating 1-methoxypropan-2-ol from an aqueous stream comprising 1-methoxypropan-2-ol and 2-methoxypropan-1-ol, wherein stream S1, which leaves distillation column B over the top, contains less than 0.05 weight-%, preferably less than 0.005 weight-%, more preferred less than 0.004 weight-%, more preferred less than 0.002 weight-%, of propylene glycol dimethyl ether, based on the total weight of S1.

A further preferred embodiment (20) concretizing any one of embodiments (1) to (19) relates to said process for separating 1-methoxypropan-2-ol from an aqueous stream comprising 1-methoxypropan-2-ol and 2-methoxypropan-1-ol, wherein stream S1 after leaving column B and before entering column C is flashed.

A further preferred embodiment (21) concretizing any one of embodiments (1) to (20) relates to said process for separating 1-methoxypropan-2-ol from an aqueous stream comprising 1-methoxypropan-2-ol and 2-methoxypropan-1-ol, wherein stream S1a comprises ≥ 95 weight-% of water, and preferably less than 1 weight-% of stream S1a consist of 1-methoxypropan-2-ol and 2-methoxypropan-1-ol, each based on the total weight of stream S1a, more preferred S1a comprises ≥ 98 weight-% of water, and less than 1 weight-% of stream S1a consist of 1-methoxypropan-2-ol and 2-methoxypropan-1-ol; more preferred S1a comprises ≥ 99 weight-% of water, and less than 1 weight-% of stream S1a consist of 1-methoxypropan-2-ol and 2-methoxypropan-1-ol.

A further preferred embodiment (22) concretizing any one of embodiments (1) to (21) relates to said process for separating 1-methoxypropan-2-ol from an aqueous stream comprising 1-methoxypropan-2-ol and 2-methoxypropan-1-ol, wherein the distillation column C comprised in the azeotropic distillation unit according to (c) has between 2 and 50 theoretical stages, preferably between 5 and 25 theoretical stages, more preferred between 10 and 20 theoretical stages.

A further preferred embodiment (23) concretizing any one of embodiments (1) to (22) relates to said process for separating 1-methoxypropan-2-ol from an aqueous stream comprising 1-methoxypropan-2-ol and 2-methoxypropan-1-ol, wherein the distillation column C comprised in the azeotropic distillation unit according to (c) is operated at a pressure in the range of from 0.5 to 10 bar, preferably in the range of from 1 to 5 bar, more preferred in the range of from 1.5 to 2.5 bar.

A further preferred embodiment (24) concretizing any one of embodiments (1) to (23) relates to said process for separating 1-methoxypropan-2-ol from an aqueous stream comprising 1-methoxypropan-2-ol and 2-methoxypropan-1-ol, wherein the distillation column C comprised in the azeotropic distillation unit according to (c) is operated at a bottoms temperature in the range of from 120 to 180 °C, preferably in the range of from 130 to 170 °C, more preferred in the range of from 140 to 160 °C.

A further preferred embodiment (25) concretizing any one of embodiments (1) to (24) relates to said process for separating 1-methoxypropan-2-ol from an aqueous stream comprising 1-methoxypropan-2-ol and 2-methoxypropan-1-ol, wherein the distillation column C comprised in the azeotropic distillation unit according to (c) is operated at a temperature at the top of the column in the range of from 70 to 110 °C, preferably in the range of from 80 to 100 °C, more preferred in the range of from 85 to 95 °C.

A further preferred embodiment (26) concretizing any one of embodiments (1) to (25) relates to said process for separating 1-methoxypropan-2-ol from an aqueous stream comprising 1-methoxypropan-2-ol and 2-methoxypropan-1-ol, wherein the distillation column C comprised in the azeotropic distillation unit according to (c) is operated with a reflux ratio in the range of from 10 to 40 g/g, preferably in the range of from 12 to 30 g/g, more preferred in the range of from 15 to 25 g/g.

A further preferred embodiment (27) concretizing any one of embodiments (1) to (26) relates to said process for separating 1-methoxypropan-2-ol from an aqueous stream comprising 1-methoxypropan-2-ol and 2-methoxypropan-1-ol, wherein the distillation column C comprised in the azeotropic distillation unit according to (c) has an energy demand in the range of from 10 to 30 MW; wherein preferably the reboiler of distillation column C has an energy demand in the range of from 5 to 15 MW and the condenser of distillation column C has an energy demand the range of from 5 to 15 MW.

A further preferred embodiment (28) concretizing any one of embodiments (1) to (27) relates to said process for separating 1-methoxypropan-2-ol from an aqueous stream comprising 1-methoxypropan-2-ol and 2-methoxypropan-1-ol, wherein the entrainer is selected from the group consisting of aromatic hydrocarbons, cyclic hydrocarbons, acyclic hydrocarbons, esters of carbonic acids, ketones, and mixtures of two or more of these compounds; preferably from the group consisting of toluene, benzene, hexane, cyclohexane, C₁-C₅ alkyl esters of carbonic acid, C₂-C₁₀ ketones and mixtures of two or more of these compounds; more preferred from the group consisting of benzene, cyclohexane, dimethyl carbonate, 2-pentanon and mixtures of two or more of these compounds; more preferred from the group consisting of benzene, cyclohexane, 2-pentanon and mixtures of two or more of these compounds; more preferred the entrainer at least comprises benzene.

A further preferred embodiment (29) concretizing any one of embodiments (1) to (28) relates to said process for separating 1-methoxypropan-2-ol from an aqueous stream comprising 1-methoxypropan-2-ol and 2-methoxypropan-1-ol, wherein the stream S2 is obtained as bottoms stream from column C, wherein ≥ 85 weight-%, preferably ≥ 90 weight-%, more preferred ≥ 95 weight-%, more preferred ≥97 weight-%, ≥ 98 weight-%, more preferred ≥ 99 weight-% of S2 consisted of 1-methoxy-2-propanol and 2-methoxy-1-propanol, based on the total weight of S2, wherein preferably stream S2 comprises 1-methoxypropan-2-ol and 2-methoxypropan-1-ol in a molar ratio in the range of from 1 : 4 to 4 : 1; preferably in the range of from 1 : 3 to 3 : 1, more preferred in the range of from 1 : 2 to 2 : 1, more preferred in the range of from 0.75 : 1 to 1 : 0.75.

A further preferred embodiment (30) concretizing any one of embodiments (1) to (29) relates to said process for separating 1-methoxypropan-2-ol from an aqueous stream comprising 1-methoxypropan-2-ol and 2-methoxypropan-1-ol, wherein the stream S2a is obtained as tom stream from distillation column C, wherein 90 weight-%, more preferred ≥ 95 weight-%, more preferred ≥97 weight-%, ≥ 98 weight-%, more preferred ≥ 99 weight-% of S2a consisted of water and the entrainer based on the total weight of S2a; wherein preferably the molar ratio of water : entrainer in the stream S2a is in the range of from 1 : 4 to 1 : 12, preferably in the range of from 1 : 6 to 1 : 10, more preferred in the range of from 1 : 8 to 1 : 9.

A further preferred embodiment (31) concretizing any one of embodiments (1) to (30) relates to said process for separating 1-methoxypropan-2-ol from an aqueous stream comprising 1-methoxypropan-2-ol and 2-methoxypropan-1-ol, wherein the distillation column D comprised in the distillation unit according to (d) has between 20 and 100 theoretical stages, preferably between 30 and 80 theoretical stages, more preferred between 40 and 60 theoretical stages, more preferred between 45 and 52 theoretical stages.

A further preferred embodiment (32) concretizing any one of embodiments (1) to (31) relates to said process for separating 1-methoxypropan-2-ol from an aqueous stream comprising 1-methoxypropan-2-ol and 2-methoxypropan-1-ol, wherein the distillation column D comprised in the distillation unit according to (d) is operated at a pressure in the range of from 0.5 to 10 bar, preferably in the range of from 1 to 5 bar, more preferred in the range of from 2 to 4 bar.

A further preferred embodiment (33) concretizing any one of embodiments (1) to (32) relates to said process for separating 1-methoxypropan-2-ol from an aqueous stream comprising 1-methoxypropan-2-ol and 2-methoxypropan-1-ol, wherein the distillation column D comprised in the distillation unit according to (d) is operated at a bottoms temperature in the range of from 140 to 200 °C, preferably in the range of from 150 to 190 °C, more preferred in the range of from 160 to 180 °C.

A further preferred embodiment (34) concretizing any one of embodiments (1) to (33) relates to said process for separating 1-methoxypropan-2-ol from an aqueous stream comprising 1-methoxypropan-2-ol and 2-methoxypropan-1-ol, wherein the distillation column D comprised in the distillation unit according to (d) is operated at a temperature at the top of the column in the range of from 120 to 200 °C, preferably in the range of from 130 to 180 °C, more preferred in the range of from 140 to 165 °C.

A further preferred embodiment (35) concretizing any one of embodiments (1) to (34) relates to said process for separating 1-methoxypropan-2-ol from an aqueous stream comprising 1-methoxypropan-2-ol and 2-methoxypropan-1-ol, wherein the distillation column D comprised in the distillation unit according to (d) is operated with a reflux ratio in the range of from 5 to 30 g/g, preferably in the range of from 8 to 20 g/g, more preferred in the range of from 10 to 15 g/g.

A further preferred embodiment (36) concretizing any one of embodiments (1) to (35) relates to said process for separating 1-methoxypropan-2-ol from an aqueous stream comprising 1-methoxypropan-2-ol and 2-methoxypropan-1-ol, wherein the distillation column D comprised in the distillation unit according to (d) has an energy demand in the range of from 1 to 5 MW; wherein preferably the reboiler of distillation column D has an energy demand in the range of from 1 to 1.5 MW and the condenser of distillation column D has an energy demand the range of from 1 to 1.5 MW.

A further preferred embodiment (37) concretizing any one of embodiments (1) to (36) relates to said process for separating 1-methoxypropan-2-ol from an aqueous stream comprising 1-methoxypropan-2-ol and 2-methoxypropan-1-ol, wherein the stream S3 is removed as top stream from distillation column D, which comprises ≥ 95 weight-% 1-methoxypropan-2-ol and ≤ 0.5 weight-% of 2-methoxypropan-1-ol, preferably ≥ 98 weight-% 1-methoxypropan-2-ol and ≤ 0.15 weight-% of 2-methoxypropan-1-ol, more preferred ≥ 99 weight-% 1-methoxypropan-2-ol and ≤ 0.1 weight-% of 2-methoxypropan-1-ol, each based on the total weight of stream S3.

A further preferred embodiment (38) concretizing any one of embodiments (1) to (37) relates to said process for separating 1-methoxypropan-2-ol from an aqueous stream comprising 1-methoxypropan-2-ol and 2-methoxypropan-1-ol, wherein the stream S3 comprises less than 0.01 weight-%, preferably less than 0.008 weight-%, more preferred less than 0.007 weight-%, more preferred less than 0.006 weight-%, of propylene glycol dimethyl ether based on the total weight of stream S3.

A further preferred embodiment (39) concretizing any one of embodiments (1) to (38) relates to said process for separating 1-methoxypropan-2-ol from an aqueous stream comprising 1-methoxypropan-2-ol and 2-methoxypropan-1-ol, wherein the stream S4 is removed as bottoms stream from distillation column D, wherein S4 comprises ≥ 95 weight-% 2-methoxypropan-1-ol and ≤ 0.5 weight-% 1-methoxypropan-2-ol, preferably ≥ 96 weight-% 2-methoxypropan-1-ol and ≤ 0.1 weight-% 1-methoxypropan-2-ol, more preferred ≥ 98 weight-% 2-methoxypropan-1-ol and ≤ 0.001 weight-% 1-methoxypropan-2-ol, more preferred ≥ 99 weight-% 2-methoxypropan-1-ol and ≤ 0.0001 weight-% 1-methoxypropan-2-ol, each based on the total weight of stream S4.

A further preferred embodiment (40) concretizing any one of embodiments (1) to (39) relates to said process for separating 1-methoxypropan-2-ol from an aqueous stream comprising 1-methoxypropan-2-ol and 2-methoxypropan-1-ol, wherein the steam stripping column E according to (e) has between 3 and 20 theoretical stages, preferably between 5 and 15 theoretical stages, more preferred between 8 and 12 theoretical stages.

A further preferred embodiment (41) concretizing any one of embodiments (1) to (40) relates to said process for separating 1-methoxypropan-2-ol from an aqueous stream comprising 1-methoxypropan-2-ol and 2-methoxypropan-1-ol, wherein the steam stripping column E according to (e) is operated at a pressure in the range of from 0.1 to 5 bar, preferably in the range of from 0.5 to 2 bar, more preferred in the range of from 0.8 to 1.2 bar.

A further preferred embodiment (42) concretizing any one of embodiments (1) to (41) relates to said process for separating 1-methoxypropan-2-ol from an aqueous stream comprising 1-methoxypropan-2-ol and 2-methoxypropan-1-ol, wherein the steam stripping column E according to (e) is operated at a temperature at the top of the distillation column B in the range of from 60 to 100 °C, preferably in the range of from 70 to 90 °C, more preferred in the range of from 75 to 85 °C.

A further preferred embodiment (43) concretizing any one of embodiments (1) to (42) relates to said process for separating 1-methoxypropan-2-ol from an aqueous stream comprising 1-methoxypropan-2-ol and 2-methoxypropan-1-ol, wherein the steam stripping column E according to (e) is operated at a temperature at the bottom in the range of from 80 to 120 °C, preferably in the range of from 85 to 115 °C, more preferred in the range of from 90 to 110 °C, more preferred in the range of from 95 to 105 °C.

A further preferred embodiment (44) concretizing any one of embodiments (1) to (43) relates to said process for separating 1-methoxypropan-2-ol from an aqueous stream comprising 1-methoxypropan-2-ol and 2-methoxypropan-1-ol, wherein the steam stripping column E according to (e) is operated with steam (H₂O_{gaseous}), which is preferably introduced into steam stripping column E in the range of from 80 to 120 kg/h, preferably in the range of from 90 to 110 kg/h, more preferred in the range of from 95 to 105 kg/h; wherein the gaseous water has preferably a pressure in the range of from 2 to 6 bar, more preferred in the range of from 3 to 5 bar, more preferred in the range of from 3.5 to 4.5 bar.

A further preferred embodiment (45) concretizing any one of embodiments (1) to (44) relates to said process for separating 1-methoxypropan-2-ol from an aqueous stream comprising 1-methoxypropan-2-ol and 2-methoxypropan-1-ol, wherein the stream S5 is preferably removed from the steam stripping column E as top stream, wherein stream S5 preferably comprises ≥ 25 weight-%, preferably ≥ 30 weight-%, entrainer.

A further preferred embodiment (46) concretizing any one of embodiments (1) to (45) relates to said process for separating 1-methoxypropan-2-ol from an aqueous stream comprising 1-methoxypropan-2-ol and 2-methoxypropan-1-ol, wherein the stream S6 is preferably removed from the steam stripping column E as bottoms stream, wherein stream S6 comprises ≥ 95 weight-% water and ≤ 1 weight-% entrainer, preferably ≥ 96 weight-% water and ≤ 0.1 weight-% entrainer, more preferred ≥ 97 weight-% water and ≤ 0.01 weight-% entrainer, more preferred ≥ 99 weight-% water and ≤ 0.0001 weight-% entrainer.

A further preferred embodiment (47) concretizing any one of embodiments (1) to (46) relates to said process for separating 1-methoxypropan-2-ol from an aqueous stream comprising 1-methoxypropan-2-ol and 2-methoxypropan-1-ol, wherein at least a part of stream S2a, after removal from distillation column C and prior to entering steam stripping column E, is introduced into a decanter, wherein at least a part pf the entrainer being contained in said part of stream S2a is separated from stream S2a obtaining a stream S2a-1 comprising entrainer and a stream S2a-2 being depleted of entrainer compared to S2a, wherein stream S2a-1 is preferably recycled to distillation column C and stream S2a-2 is introduced into steam stripping column E.

A further preferred embodiment (48) concretizing any one of embodiments (1) to (47) relates to said process for separating 1-methoxypropan-2-ol from an aqueous stream comprising 1-methoxypropan-2-ol and 2-methoxypropan-1-ol, wherein the process, preferably if the methanol content in stream S0 is > 0.1 weight-%, more preferred in the range of from 0.02 to 1.00 weight-%, each based on the total weight of stream S0, further comprises the step:
f) separating methanol from stream S1 by distillation, comprising subjecting the stream S1 obtained in (b) to distillation conditions in a distillation unit comprising a distillation column F, obtaining a stream S1' comprising 1-methoxypropan-2-ol, 2-methoxypropan-1-ol and ≤ 0.1 weight-% methanol based on the total weight of S1', wherein S1' is introduced in step (c) into extractive column C instead of stream S1;
   or
g) separating methanol from stream S6 by distillation, comprising subjecting the stream S6 obtained in (d) to distillation conditions in a distillation unit comprising a distillation column F, obtaining a stream S6', which comprises ≥ 95 weight-% water, ≤ 1 weight-% entrainer and ≤ 0.1 weight-% methanol, preferably ≥ 96 weight-% water, ≤ 0.1 weight-% entrainer and ≤ 0.1 weight-% methanol;
wherein preferably step (g) is used.

A further preferred embodiment (49) concretizing any one of embodiments (1) to (48) relates to said process for separating 1-methoxypropan-2-ol from an aqueous stream comprising 1-methoxypropan-2-ol and 2-methoxypropan-1-ol, wherein distillation column F comprises in the range of from 5 to 25 theoretical stages, preferably in the range of from 7 to 15 theoretical stages, more preferred in the range of from 8 to 12 theoretical stages; and/or wherein distillation column F is operated at a pressure in the range of from 0.5 to 1.5 bar, preferably in the range of from 0.8 to 1.2 bar; and/or wherein distillation column F has an energy demand in the range of from 1 to 3 MW; preferably an energy demand in the condenser in the range of from 0.5 to 1.5 MW and/or an energy demand in the range of from 0.5 to 1.5 MW in the reboiler.

It is explicitly noted that the preceding set of embodiments is not the set of claims determining the extent of protection, but represents a suitably structured part of the description directed to general and preferred aspects of the present invention.

The present invention is further illustrated by the following reference examples, comparative examples, and examples.

### Examples

### Simulations

All simulations were done with process simulation software Aspen Plus v.11. The components used in the process simulation and their characteristics respectively, were taken from the Dortmund Database.

### Example 1: separation of 1-methoxypropan-2-ol from an aqueous stream (S0) containing 85 weight-% of water and 14.2 weight-% of a mixture of 2-methoxypropan-1-ol and 1-methoxypropan-2-ol

The feed stream S0 to column B was a variable stream and represented a stream from a propylene oxide production process, wherein a reaction mixture comprising propylene, water, methanol, and hydrogen peroxide had been contacted in an epoxidation zone with an epoxidation catalyst comprising a zeolitic material having a framework structure comprising Si, O, and Ti and being of framework type MFI (titanium silicalite-1 (TS-1)), and subjecting the reaction mixture to epoxidation reaction conditions in the epoxidation zone.

The obtained mixture comprising propylene oxide, water, and methanol had been removed as an effluent stream from the epoxidation zone. The effluent stream comprising propylene oxide, water, and methanol had been subjected to further separation and purification steps, wherein propylene oxide and water as well as parts of the organic solvent had been removed, resulting in a stream S0 comprising 1-methoxypropan-2-ol, 2-methoxypropan-1-ol and water and having a molar ratio of 1-methoxypropan-2-ol : 2-methoxypropan-1-ol in the range of from 1 : 5 to 5 : 1. The composition of stream S1 varied as a function of the operating conditions of the propylene oxide production process (see influence of feed stream on the separation in Examples 2 and 3). An exemplary composition of stream S0 and exemplary compositions of the further streams S1 to S6 are indicated in Table 1; stream S0 as indicated in Table 1 of Example 1 contained 85 weight-% of water, 14.2 weight-% 2-methoxypropan-1-ol and 1-methoxypropan-2-ol and 0.02 weight-% methanol (MeOH).

**Table 1**

| Compositions and parameters of streams S0 to S6, including benzene stream to column C and steam stream to column E | | | | | | | | | | | |
|---|---|---|---|---|---|---|---|---|---|---|---|
| Streams | **S0** | **S1a** | **S1** | **Benzene** (stream directed to column C | **S2** | **S2a** | **S5** | **S6** | **Steam** (H₂O (g), stream directed to column E | **S3** | **S4** |
| Parameter | | | | | | | | | | | |
| Temperature [°C] | 181 | 179.927 | 175.099 | 20 | 147.994 | 86.9 | 25 | 99.39 | 143 | 154.87 | 172.10 |
| Pressure [bar] | 10 | 10 | 10 | 2 | 2 | 2 | 1 | 1 | 1 | 3 | 3 |
| Mass flow [kg/h] | 11415 | 7371 | 4044 | 4.189 | 1623 | 23798 | 21373.61 | 2519 | 95 | 792.77 | 829.79 |

| Components, indicated in weight-% | | | | | | | | | | | |
|---|---|---|---|---|---|---|---|---|---|---|---|
| **2-Methoxypropan-1-ol** | **7.26** | **5.83E-04** | **20.48** | | **51.11** | **2.8E-05** | **8.06E-06** | **2.31E-05** | | **5E-02** | **99.87** |
| Water | 85.17 | 99.11 | 59.98 | | 1E-02 | 10.56 | 22.43 | 99.86 | 1 | 2.01E-02 | |
| Benzene | | | | 1 | 4.67E-08 | 86.73 | 34.16 | | | 9.49E-08 | |
| **1-Methoxypropan-2-ol** | **6.95** | **9.42E-03** | **19.46** | | **48.83** | **1E-02** | **4.44E-03** | **6.52E-03** | | **99.92** | **3E-02** |
| 1,1-Dimethoxyethane | | | | | | | | | | | |
| 1,1-Dimethoxypropane | 1E-03 | | 1.34E-03 | | 3.05E-08 | 1.5 | 4.84 | 2.92E-09 | | 6.15E-08 | |
| 1,2-Propanediol (MPG) | 1E-02 | 1.55E-2 | 9.45E-8 | | 2.22E-07 | | | | | | 4.34E-07 |
| 1-Butanol | 1E-03 | | 2.27E-03 | | 1.84E-03 | 1.16E-02 | 1.94E-02 | 3.33E-03 | | 3.77E-03 | 2.87E-08 |
| 2,4-Dimethyl-1,3-dioxolane | 1.E-03 | | 1.81E-03 | | 3.9E-08 | 1.75E-01 | 2.39 | 4.02E-03 | | 7.87E-08 | |
| 2,6-Dimethyl-4-heptanol | 1E-03 | | 1.71E-03 | | 7.04E-03 | | | | | | 1.38E-02 |
| 2-Butenal | 1E-03 | | 2.22E-03 | | 4.9E-04 | 3.3E-02 | 3.82E-02 | 4.19E-03 | | 9.99E-04 | 2.04E-13 |
| 2-Ethyl-4-methyl-1,3-dioxolane | 1E-03 | 1.55E-03 | 5.2E-06 | | 1.24E-05 | | | | | 7.36E-08 | 2.41E-05 |
| 2-Hexanone | 1E-01 | | 1.56E-01 | | 7.04E-01 | 2.38E-02 | 2.43E-02 | 1.15E-08 | | 4.19E-03 | 1.37 |
| 2-Methylcyclohexanol | 1E-03 | 1.55E-03 | 5.2E-06 | | 1.24E-05 | | | | | 7.36E-08 | 2.41E-05 |
| 2-Methylpentanal | 4E-03 | | 4.85E-03 | | 2.81E-02 | 7.82E-03 | 2.71E-03 | 3.06E-14 | | 1.68E-04 | 5.48E-02 |
| 2-Propen-1-ol | 1E-03 | | 2.51E-03 | | 3.26E-06 | 2.67E-03 | 1.14E-02 | 4.53E-03 | | 6.64E-06 | |
| 4-Methyl-1,3-dioxolane | 1E-03 | 3.53E-10 | 2.76E-03 | | 7.03E-03 | 1.63E-06 | 6.9E-07 | 5.13E-07 | | 4.19E-05 | 1.37E-02 |
| Acetaldehyde | 1E-0 | | 1.46E-03 | | 1.27E-10 | 1.85E-01 | 1.32E-01 | 1.56E-03 | | 1.99E-11 | |
| Acetone | 1E-03 | | 2.06E-03 | | 1.5E-08 | 7.51E-03 | 5.71E-02 | 4.5E-03 | | 3.01E-08 | |
| Dimethoxymethane | 1E-03 | | 1.99E-03 | | 4.52E-09 | 3.7E-01 | 1.16E-01 | 1.69E-03 | | 9.04E-09 | |
| Dipropyleneglycol (DPG) | 2E-02 | 3.1E-02 | 1.04E-04 | | 2.47E-04 | | | | | 1.47E-06 | 4.82E-04 |
| Ethanol | 1E-03 | | 2.28E-03 | | 6.37E-08 | 1.52E-03 | 1.32E-02 | 4.53E-03 | | 1.28E-07 | |
| Hydroxyacetone | 1E-03 | 1.51E-03 | 7.55E-05 | | 1.81E-04 | | | | | 2.33E-12 | 3.55E-04 |
| 2-Propanol | 1E-03 | | 2.03E-03 | | 1.33E-07 | 3.1E-03 | 2.71E-02 | 4.52E-03 | | 2.68E-07 | |
| Methanol | 1.99E-02 | | 4.84E-02 | | 1.58E-07 | 1.54E-02 | 1.82E-01 | 9E-02 | | 3.16E-07 | |
| Methylacetate | 1E-03 | | 1.68E-03 | | 1.11E-08 | 1.11E-01 | 2.54E-02 | 4.02E-03 | | 2.23E-08 | |
| Methylformate | 1E-03 | 1.54E-03 | 1.81E-05 | | 3.12E-12 | 7.14E-05 | 2.31E-03 | 2.7E-05 | | | |
| Propyleneoxide | 1E-03 | | 1.59E-03 | | 5.57E-10 | 2.63E-01 | 8.46E-02 | 2.63E-03 | | 1.1E-09 | |
| Tripropylene glycol (TPG) | 2E-02 | 3.1E-02 | 7.75E-10 | | 6.97E-12 | | | | | | |
| Dipropylene glycol mono methyl ether (DPGME) | 5.1E-01 | 7.9E-01 | 4.63E-06 | | 1.09E-05 | | | | | | 2.13E-05 |
| **Propylene glycol dimethyl ether (1,2-Dimethoxypropane)** | **6.5E-03** | **9.23E-03** | **1.56E-03** | | **2.71E-03** | **2.4E-04** | **2.06E-04** | **7.01E-04** | | **5.52E-03** | **6.93E-14** |

The expression "E-xx" in Table 1 represents 10^{-xx}, wherein "xx" is here a placeholder for the respective number indicated in Table 1.

Column B was a pre-distillation column, used to enrich the mixture of 1-methoxy-2-propanol and 2-methoxy-1-propanol contained in stream S0. In column B, the mixture of 1-methoxy-2-propanol and 2-methoxy-1-propanol was separated from side components. in order to ensure that the final product 1-methoxy-2-propanol has a purity of > 95 weight-%, preferably ≥ 98 weight-%, more preferred ≥ 99 weight-%, more preferred ≥ 99.7 weight-%. Column B had 20 theoretical stages and was operated at 10 bar. Feed stream S0 entered the column B at theoretical stage 17 (between stage 17 and 18). The temperature at the top was 177 °C and at the bottom 180 °C. Column B was operated with a reflux ratio of 4.93 g/g and 9.17 MW were needed in the reboiler and 9.186 MW in the condenser. An azeotropic mixture of water, 1-methoxy-2-propanol and 2-methoxy-1-propanol was removed from column B over the top (stream S1: 60 weight-% water, 40 weight-% mixture of 1-methoxy-2-propanol and 2-methoxy-1-propanol). Negligible amounts of side components were removed from column B as bottoms stream S1a, which was afterwards send to a subsequent water treatment.

Stream S1 was optionally flashed before entering column C to reduce the heat demand in column C, wherein the stream S1 was transferred to a further flash column, where the pressure was reduced from 10 bar to 2 bar. This decreased the temperature of stream S1, which in turn reduced the heat demand in column C.

Column C was an azeotropic distillation column working with benzene as entraining agent with 16 theoretical stages operated at 2 bar. The temperature at the top was 89.5 °C and at the bottom 148 °C. Column C was operated with a reflux ratio of 18.12 g/g and 7.38 MW were needed in the reboiler and 7.7 MW in the condenser. As entrainer in Column B, benzene was used. A bottom streams S2 was removed from Column C, wherein ≥ 85 weight-% of S2 consisted of 1-methoxy-2-propanol and 2-methoxy-1-propanol. A stream S2a consisting to more than 90 weight-% of water and benzene was removed from column C over the top and further proceeded in column E.

Stream S2 was transferred to column D, which was a distillation column with 48 theoretical stages operated at 3 bar. The temperature at the top of column D 156 °C and at the bottom 172 °C. Column D was operated with a reflux ratio of 12.2 g/g and 1.245 MW were needed in the reboiler and 1.23 MW in the condenser. From column D, a stream S3 was removed over the top comprising ≥ 95 weight-% 1-methoxypropan-2-ol and ≤ 0.5 weight-% of 2-methoxypropan-1-ol (as in Table 1 indicated, 0.05% 2-methoxypropanol in S3), based on the total weight of stream S3. As bottoms streams, a stream S4 was removed, wherein S4 comprised ≥ 95 weight-% 2-methoxypropan-1-ol based on the total weight of stream S4 (as in Table 1 indicated, 300 ppm of 1-methoxypropanol in S4).

Column E was used to recover the benzene- via steam stripping. Column E was a steam stripping column with 10 theoretical stages operated at 1 bar. A stream of 98 kg/h 4 bar steam (H₂O_{gaseous}) was used as the stripping agent to separate completely the water from the entrainer, avoiding any agent losses and assuring the water specifications before water treatment. The temperature at the top of column E was 81.7 °C and at the bottom 99.4 °C. Stream S6 which left column E as bottoms stream was cooled down to about 25 ° before subsequent water treatment and did not contain any entrainer.

### Heat integration

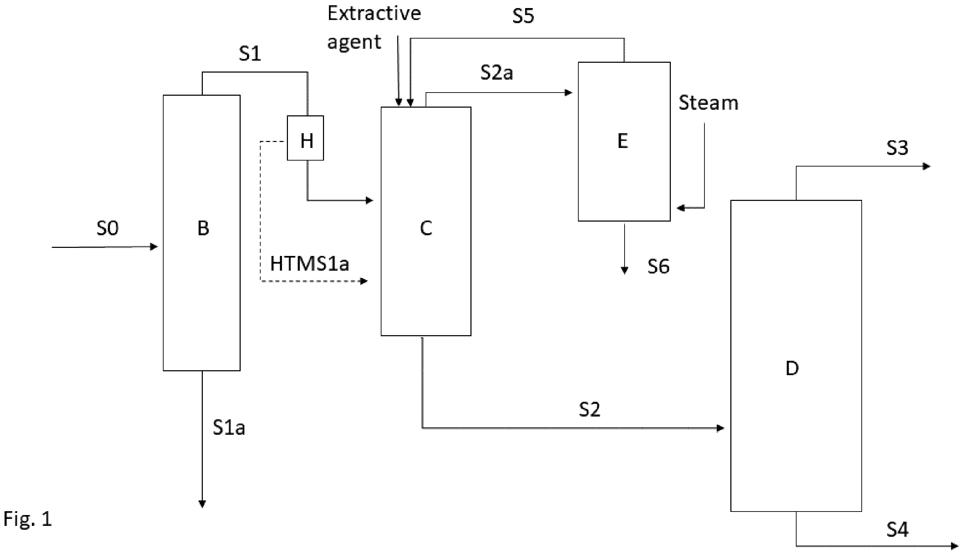

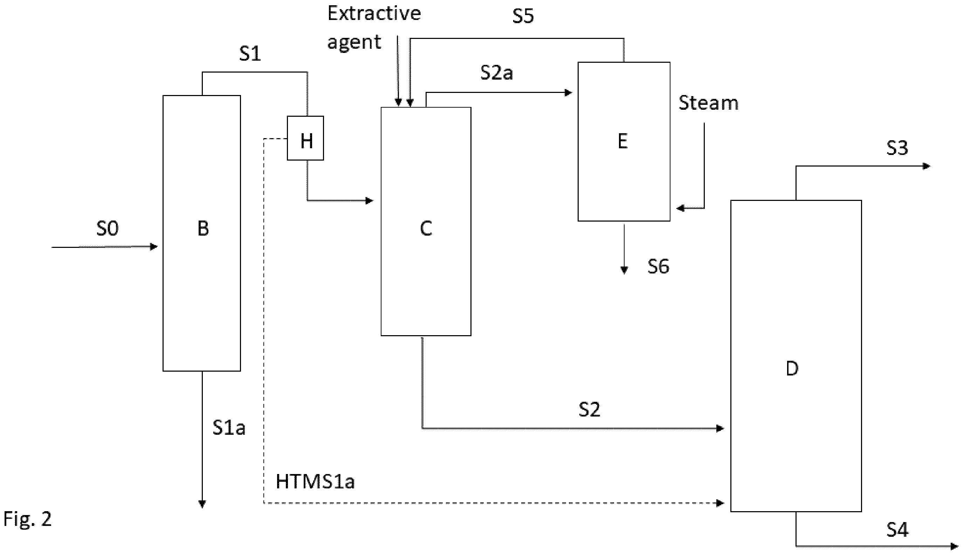

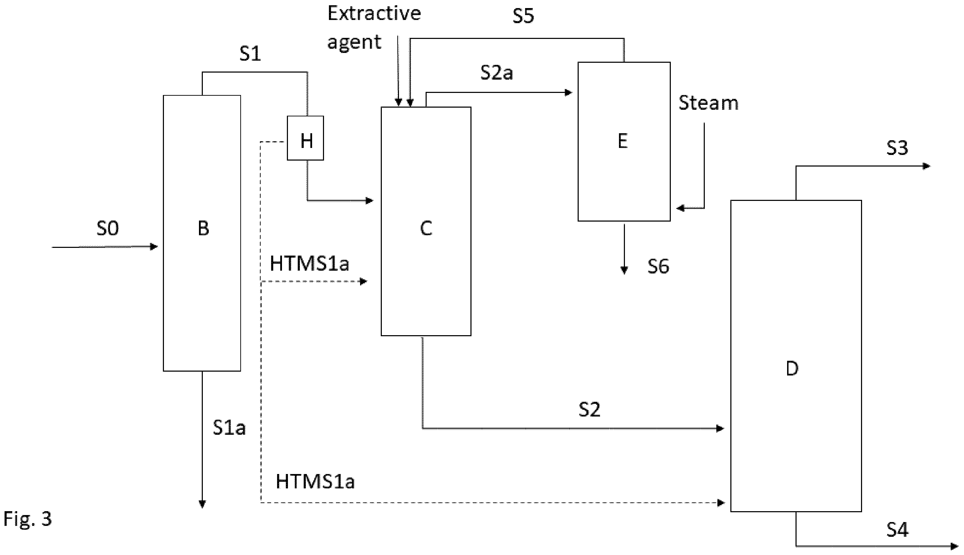

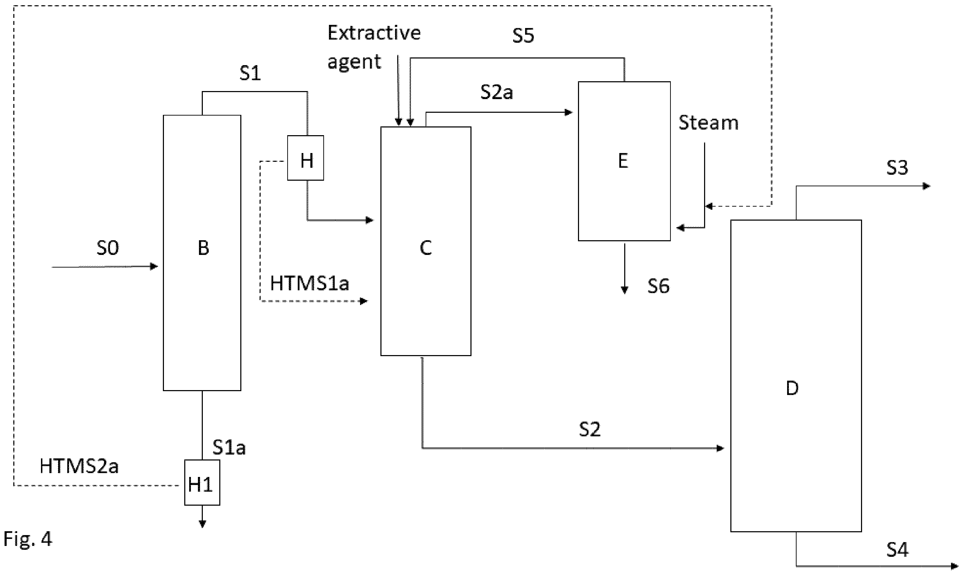

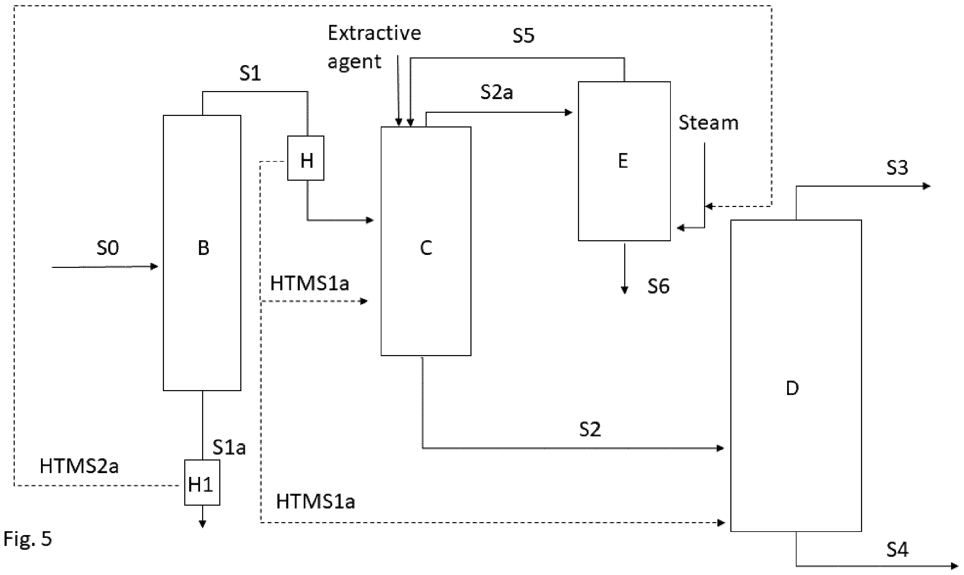

An important aspect of the separation process in including columns B to E and optionally F was the heat integration, because it reduces significantly the investment costs in terms of steam. For the heat integration there were several possibilities, 5 thereof were simulated:
1. The thermal energy (heat) of the condenser of column B, i.e. of stream S1, was used to heat the reboiler of column C in that thermal energy of stream S1 was partly transferred to a heat transfer medium stream HTMS1 after step (b) and before step (c) in a heat exchanger H, wherein a heat transfer medium stream HTMS1a was obtained which had an increased thermal energy content compared to HTMS1. The heat transfer medium stream HTMS1a was used to provide thermal energy to a heat exchanger unit, which supplied the reboiler of column C (see Fig. 1). For example, water from the condenser of column B was used to heat up the heat exchanger unit supplying the reboiler of column C. The heat of the condenser of column B, i.e. the partial amount of thermal energy taken from stream S1, was 9.18 MW, wherefrom 7.38 MW were used to heat up the reboiler of column C - no further energy input was needed for the reboiler of column C.
2. The thermal energy (heat) of the condenser of column B, i.e. of stream S1, was used to heat the reboiler of column D in that thermal energy of stream S1 was partly transferred to a heat transfer medium stream HTMS1 after step (b) and before step (c) in a heat exchanger H, wherein a heat transfer medium stream HTMS1a was obtained which had an increased thermal energy content compared to HTMS1. The heat transfer medium stream HTMS1a was used to provide thermal energy to a heat exchanger unit, which supplied the reboiler of column D (see Fig. 2). The heat of the condenser of column B, i.e. the partial amount of thermal energy taken from stream S1, was 9.18 MW, wherefrom 1.24 MW were used to heat up the reboiler of column D - no further energy input was needed for the reboiler of column D.
3. The thermal energy (heat) of the condenser of column B, i.e. of stream S1, was used to heat the reboiler of columns C and D in that thermal energy of stream S1 was partly transferred to a heat transfer medium stream HTMS1 after step (b) and before step (c) in a heat exchanger H, wherein a heat transfer medium stream HTMS1a was obtained which had an increased thermal energy content compared to HTMS1. The heat transfer medium stream HTMS1a was used to provide thermal energy to a heat exchanger unit, which supplied the reboiler of column C and to heat exchanger unit, which supplied the reboiler of column D (see Fig. 3). The heat of the condenser of column B, i.e. the partial amount of thermal energy taken from stream S1, was 9.18 MW, wherefrom 7.38 MW were used to heat up the reboiler of column C and 1.24 MW were used to heat up the reboiler of column D - no further energy input was needed for the reboilers of columns C and D.
4. The thermal energy of stream S1a, due to its high temperature, was used to produce 4 bar steam (gaseous H₂O) with a rate of 521 kg/h, being only 98 k/h needed in column E for the steam stripping in that thermal energy of stream S1a was partly transferred to a heat transfer medium stream HTMS2 after step (b) in a heat exchanger H1, wherein a heat transfer medium stream HTMS2a was obtained which had an increased thermal energy content compared to HTMS2. HTMS2a was then used as the steam required for column E (see Fig. 4). No further energy input was required for generating the steam for column E. The thermal energy (heat) of the condenser of column B, i.e. of stream S1, was used to heat the reboiler of column C in that thermal energy of stream S1 was partly transferred to a heat transfer medium stream HTMS1 after step (b) and before step (c) in a heat exchanger H, wherein a heat transfer medium stream HTMS1a was obtained which had an increased thermal energy content compared to HTMS1. The heat transfer medium stream HTMS1a was used to provide thermal energy to a heat exchanger unit, which supplied the reboiler of column C.
   The power required for the separation was just 9.6 MW or 7.68 MW·h/t of pure methoxypropanols (1250 kg/h of pure methoxypropanols).
5. as shown in Fig. 5, a complete heat integration was made. The thermal energy (heat) of the condenser of column B, i.e. of stream S1, was used to heat the reboiler of columns C and D in that thermal energy of stream S1 was partly transferred to a heat transfer medium stream HTMS1 after step (b) and before step (c) in a heat exchanger H, wherein a heat transfer medium stream HTMS1a was obtained which had an increased thermal energy content compared to HTMS1. The heat transfer medium stream HTMS1a was used to provide thermal energy to a heat exchanger unit, which supplied the reboiler of column C and to heat exchanger unit, which supplied the reboiler of column D. The heat of the condenser of column B, i.e. the partial amount of thermal energy taken from stream S1, was 9.18 MW, wherefrom 7.38 MW were used to heat up the reboiler of column C and 1.24 MW were used to heat up the reboiler of column D. Further, the thermal energy of stream S1a was used to produce the steam needed in column E in that thermal energy of stream S1a was partly transferred to a heat transfer medium stream HTMS2 after step (b) in a heat exchanger H1, wherein a heat transfer medium stream HTMS2a was obtained which had an increased thermal energy content compared to HTMS2. HTMS2a was then used to produce the steam required for column E. The remaining energy demand for the complete process of columns B to E was only 8.9 MW or 7.12 MW·h/t of pure methoxypropanols.

The heat transfer medium of HTMS1/HTMS1a and of HTMS2/HTMS2a was steam (H₂O_{gaseous}).

This example demonstrated that 1-methoxypropanol-2 with a purity of 99.92 weight-% could be obtained with an isolation yield of 99.84%. Additionally, 2-methoxypropanol-1 with a purity of 99.87 weight-% could be obtained with an isolation yield of 99.99%. The power required for the separation was in the best constellation just 8.9 MW or 7.12 MW·h/t of pure methoxypropanols (1250 kg/h of pure methoxypropanols).

### Example 2: influence of the MeOH concentration in the feed stream SO on the separation of 1-methoxy-2-propanol

Herein, the same set-up with the same columns B to E as in Example 1 was used, the only difference to Example 1 was that in stream S0 the amount of MeOH contained in the stream was assumed to be 0.1 weight-%.

Herein, the same set-up with the same columns B to E as in Example 1 was used, the only difference to Example 1 was that in stream S0 the amount of MeOH contained in the stream was assumed to be 0.1 weight-%.

The simulations were made according to a value of 0.1 weight-% MeOH, resulting in no presence of MeOH in the water streams that have to be send to a subsequent water treatment (streams S1a, S6).

In case of presence of more MeOH in the stream S0 (assumed here is an extreme value of 1 weight-% MeOH),-simulations were performed, indicating that the MeOH would go completely to the feed stream of column C (S1) and it would be an increase from 0.048 weight-% in Example 1 (0.1 weight-% MeOH in S0) to 2.76 weight-% in Example 4 (1 weight-% MeOH in S0). The MeOH would go completely to the water stream (S6), 114.15 kg/h MeOH in S0 that go to S6 with 113.984 and 0.116 to S5 back into column C.

There were two possibilities to operate in case of 1 weight-% MeOH in S0: first, to separate the MeOH before entering the column C, or second, to separate it from stream S6 before going to a subsequent water treatment. For both possibilities, the MeOH can be to 100 weight-% separated from the water with a further distillation column F, needing 1.07 MW in the condenser and 1.09 MW in the reboiler, 10 theoretical stages and 1 bar.

The heat transfer medium of HTMS1/HTMS1a and of HTMS2/HTMS2a was steam (H₂O_{gaseous}).

This example demonstrated that in S3, 1-methoxypropanol-2 with a purity of 99.92 weight-% could be obtained with an isolation yield of 99.22%. Additionally, 2-methoxypropanol-1 with a purity of 99.87 weight-% could be obtained in S4 with an isolation yield of 99.94%. The power required for the separation was just 10.97 MW or 8.77MW·h/t of pure methoxypropanols (1250 kg/h methoxypropanols).

Table 2 lists streams S0, S1, S1a, S2, S2a and S5 for the composition with 1 weight-% MeOH in S0.

**Table 2**

| streams for a composition of S0 with 1 weight-% MeOH | | | | | | | |
|---|---|---|---|---|---|---|---|
| Streams | **S0** | **S1a** | **S1** | **Benzene** (stream directed to column (c) | **S2** | **S2a** | **S6** |
| Temperature [°C] | 181 | 179.93 | 177.47 | 20 | 147.88 | 83.7 | 25 |
| Pressure [bar] | 10 | 10 | 10 | 2 | 2 | 2 | 1 |
| Mass flow [kg/h] | 11415 | 7290 | 4125 | 3.7 | 1623 | 25100 | 22595 |

| Components, indicated in weight-% (unless otherwise noted) | | | | | | | |
|---|---|---|---|---|---|---|---|
| 2-Methoxypropan-1-ol | 7.26 | 6E-04 | 20.16 | | 51.11 | 2.8E-05 | 281 ppb |
| Water | 84.19 | 99.11 | 58.22 | | 1E-02 | 10.31 | 0.44 |
| Benzene | | | | 1 | 0 | 84.92 | 94.89 |
| 1-Methoxypropan-2-ol | 6.95 | 9.4E-03 | 19.13 | | 48.83 | 1E-02 | 1.03E-03 |
| 1,1-Dimethoxyethane | | | | | | | |
| 1,1-Dimethoxypropane | 1E-03 | | 1.0E-03 | | | 1.58 | 1.76 |
| 1,2-Propanediol (MPG) | 1E-02 | 1.55E-2 | 1 ppb | | 2.0E-06 | | |
| 1-Butanol | 1E-03 | | 2.22E-03 | | 1.8E-03 | 1.14E-02 | 2.88E-2 |
| 2,4-Dimethyl-1,3-dioxolane | 1.E-03 | | 1.6E-03 | | | 0.63 | 0.69 |
| 2,6-Dimethyl-4-heptanol | 1E-03 | | 1.5E-03 | | 7.0E-03 | | |
| 2-Butenal | 1E-03 | | 2.1E-03 | | 5.0E-4 | 3.24E-02 | 3.73E-03 |
| 2-Ethyl-4-methyl-1,3-dioxolane | 1E-03 | 1.5E-03 | 5.2E-06 | | 1.22E-05 | | |
| 2-Hexanone | 1E-01 | | 1.3E-03 | | 7.0E-03 | 2.0 E-03 | 2.0E-04 |
| 2-Methylcyclohexanol | 1E-03 | 1.5E-03 | 5.2E-06 | | 1.22E-05 | | |
| 2-Methylpentanal | 4E-03 | | 3.6E-03 | | 2.81E-02 | 7.9E-03 | 2.6E-03 |
| 2-Propen-1-ol | 1E-03 | | 2.51E-03 | | 3.2E-06 | 2.7E-03 | 1.13E-02 |
| 4-Methyl-1,3-dioxolane | 1E-03 | | 2.7 E-03 | | 7.0E-03 | 1.6E-06 | 7 ppb |
| Acetaldehyde | 1E-0 | | 1.2E-03 | | | 2E-01 | .0.2 |
| Acetone | 1E-03 | | 1.9E-03 | | | 7.5E-03 | 5.57E-2 |
| Dimethoxymethane | 1E-03 | | 1.8E-03 | | | 5.5E-01 | 0.62 |
| Dipropyleneglycol (DPG) | 2E-02 | 3.1E-02 | 1.0E-04 | | 2.0E-04 | | |
| Ethanol | 1E-03 | | 2.3E-03 | | 1ppb | 1.5E-03 | 1.3E-03 |
| Hydroxyacetone | 1E-03 | 1.5E-03 | 1E-04 | | 2.0E-04 | | |
| 2-Propanol | 1E-03 | | 1.9E-03 | | 1ppb | 3.1E-03 | 2.67E-03 |
| Methanol | 1 | | 2.76 | | 75 ppb | 4.71E-01 | 5.34E-04 |
| Methylacetate | 1E-03 | | 1.5E-03 | | | 3.6E-01 | 2.92 |
| Methylformate | 1E-03 | 1.5E-03 | 164 ppb | | | 1.0E-04 | 2.6 E-03 |
| Propyleneoxide | 1E-03 | | 1.7E-03 | | | 5.4E-01 | 0.58 |
| Tripropyleneglycol (TPG) | 2E-02 | 3.1E-02 | 0 | | | | |
| Dipropyleneglycol monomethyl ether (DPGME) | 5.1E-01 | 7.9E-01 | 41 ppb | | 1.08E-05 | | |
| Propylene glycol dimethyl ether (1,2-Dimethoxypropane) | 6.5E-03 | 9.2E-03 | 1.4E-03 | | 2.7E-03 | 2.0E-04 | 2.0E-04 |

### Example 3: influence of the water concentration in the feed stream S0 on the separation of 1-methoxy-2-propanol

Herein, the same set-up with the same columns B to E as in Example 1 was used, the only difference to Example 1 was that in stream S0 a different water concentration was simulated to investigate its influence on the separation of 2-methoxypropan-1-ol and 1-methoxypropan-2-ol. From a composition with 85 weight-% water and 14.2 weight-% of 2-methoxypropan-1-ol and 1-methoxypropan-2-ol up to a composition with 55 weight-% H₂O and 43 weight-% 2-methoxypropan-1-ol and 1-methoxypropan-2-ol, the separation could be done successfully as described in Example 1. The amount of methoxypropanols in the feed was higher in Example 3, therefore the amount over the top in column B was higher because there was more amount of the azeotrope water/methoxypropanols. Therefore, a bit more amount of fresh benzene was needed to break the azeotrope in column C, 6 kg/h instead of 4.2 kg/h in Example 1 (see Table 3).

In Column E, 200 kg/h of steam were required instead of only 95 kg/h as in Example 1.

The stream containing the 2 MOP isomers, S2, contained according to the specifications, 100 ppm water. A stream S0 with less water than 55 weight-% and/ or more than 43 weight-% 2-methoxypropan-1-ol and 1-methoxypropan-2-ol would not be possible because it would be under the azeotrope point. Table 3 lists streams S0, S1, S1a, S2, S2a and S5 for the composition with 55 weight-% H₂O and 43 weight-% 2-methoxypropan-1-ol and 1-methoxypropan-2-ol.

**Table 3**

| streams for a composition of S0 with 55 weight-% H₂O and 43 weight-% 2-methoxypropan-1-ol and 1-methoxypropan-2-ol. | | | | | | | |
|---|---|---|---|---|---|---|---|
| Streams | **S0** | **S1a** | **S1** | **Benzene** (stream directed to column (c) | **S2** | **S2a** | **S5** |
| Temperature [°C] | 181 | 192.32 | 177.47 | 20 | 147.88 | 87.82 | 25 |
| Pressure [bar] | 10 | 10 | 10 | 2 | 2 | 2 | 1 |
| Mass flow [kg/h] | 11415 | 86.79 | 11328.21 | 6 | 5048 | 62372 | 56086 |
| | | | | | | | |

| Components, indicated in weight-% (unless otherwise noted) | | | | | | | |
|---|---|---|---|---|---|---|---|
| 2-Methoxypropan-1-ol | 22.26 | 4.87E-03 | 22.43 | | 50.360 | 356 ppb | 3.62E-05 |
| Water | 55.17 | 25.25 | 55.41 | | 100 ppm | 10.75 | 0.41 |
| Benzen | | | | 1 | 1E-06 | 89.12 | 99.46 |
| 1-Methoxypropan-2-ol | 21.95 | 5.13E-03 | 22.11 | | 49.61 | 1E-02 | 1.04E-03 |
| 1,1-Dimethoxyethane | | | | | | | |
| 1,1-Dimethoxypropane | 1E-03 | | 1E-03 | | | 0.055 | 6.94E-02 |
| 1,2-Propanediol (MPG) | 1E-02 | 1.32 | 2.09E-08 | | 1 ppb | | |
| 1-Butanol | 1E-03 | | 1E-03 | | 6E-04 | 4E-03 | 4.32E-03 |
| 2,4-Dimethyl-1,3-dioxolane | 1.E-03 | | 1.E-03 | | | 1.15E-02 | 1.26E=-02 |
| 2,6-Dimethyl-4-heptanol | 1E-03 | | 1E-03 | | 2.3E-03 | | |
| 2-Butenal | 1E-03 | | 1E-03 | | 2E-04 | 1.15E-02 | 1.27E-03 |
| 2-Ethyl-4-methyl-1,3-dioxolane | 1E-03 | 1.31E-01 | 1.21E-06 | | 54 ppb | | |
| 2-Hexanone | 1E-03 | | 1E-03 | | 2.3E-03 | 1E-04 | 5.75E-05 |
| 2-Methylcyclohexanol | 1E-03 | 1.31E-01 | 1.21E-06 | | 54 ppb | | |
| 2-Methylpentanal | 4E-03 | | 4E-03 | | 9E-03 | 1.5E-03 | 1.71E-03 |
| 2-Propen-1-ol | 1E-03 | | 2.51E-05 | | 15 ppb | 1E-03 | 8.6E-04 |
| 4-Methyl-1,3-dioxolane | 1E-03 | 3.36E-09 | 1.01E-03 | | 2.3E-03 | 5E-06 | 6E-07 |
| Acetaldehyde | 1E-0 | | 1.01E-03 | | | 5.8E-03 | 2.67E-03 |
| Acetone | 1E-03 | | 1.01E-03 | | | 2.6E-03 | 2.67E-03 |
| Dimethoxymethane | 1E-03 | | 1.01E-03 | | 1E-06 | 5.8E-03 | 6.3E-03 |
| Dipropyleneglycol | 2E-02 | 2.68 | 2.42E-05 | | 1E-05 | | |
| Ethanol | 1E-03 | | 1.01E-03 | | | 6E-04 | 4E-04 |
| Hydroxyacetone | 1E-03 | 1.3E-01 | 9.03E-06 | | 374 ppb | | |
| 2-Propanol | 1E-03 | | 1.01E-03 | | 1 ppb | 1.1E-03 | 1E-03 |
| Methanol | 1.99E-02 | | 2.0E-02 | | 1 ppb | 5.8E-03 | 2.3E-03 |
| Methylacetate | 1E-03 | | 1E-03 | | | 7E-03 | 7.6E-03 |
| Methylformate | 1E-03 | 1.31E-01 | 2.6E-06 | | | 1.24E-05 | 128 ppb |
| Propyleneoxide | 1E-03 | | 1E-03 | | | 5.6E-03 | 6E-03 |
| Tripropyleneglycol (TPG) | 2E-02 | 2.63 | 4.07E-11 | | | | |
| Dipropyleneglycol monomethylether (DPGME) | 5.1E-01 | 67 | 1.03E-06 | | 48 ppb | | |
| Propylene glycol dimethyl ether (1,2-Dimethoxypropane) | 6.5E-03 | 5.7E-01 | 2.18E-03 | | 3.93E-03 | 5E-04 | 4E-04 |

From Examples 1 to 3, as well as from Tables 1 to 3, it could be seen that operating column B at a pressure ≥ 2 bar (here 10 bar), resulted in a separation of propylene glycol dimethyl ether: stream S1, after column B had only a content of propylene glycol dimethyl ether of less than 0.06 weight-%, preferably less than 0.05 weight-%, more preferred less than 0.005 weight-%, more preferred less than 0.004 weight-%, more preferred less than 0.002 weight-%, of propylene glycol dimethyl ether, based on the total weight of S1.

The example demonstrated that 1-methoxypropanol-2 with a purity of 99.92 weight-% could be obtained as S3 with an isolation yield of 99.86%. Additionally, 2-methoxypropanol-1 with a purity of 99.94 weight-% could be obtained as S4 with an isolation yield of 99.91%. Especially stream S3, which contained 1-methoxypropanol-2, had less than 0.06 weight-%, preferably less than 0.01 weight-%, more preferred less than 0.008 weight-%, more preferred less than 0.007 weight-%, more preferred less than 0.008 weight-%, of propylene glycol dimethyl ether based on the total weight of stream S3.

The power required for the separation was 16.64 MW, or 3.3 MW·h/t of pure methoxypropanols (5046 kg/h pure methoxypropanols). Further, it could be seen that also other impurities in the final products were significantly reduced when column B was operated at a pressure ≥ 2 bar.

### Example 4

Example 1 was recalculated but the pressure in column B was reduced to 3.5 bar while keeping all the other parameters constant.

At lower pressure it was still possible to energetically couple the towers, but not as efficiently, causing the energy consumption to increase to 10 MW which is almost 5% higher than in example 1. Surprisingly the separation of by-products at lower pressure is also less efficient. Table 4 shows that the concentration of several by-products in stream S1 is considerably higher than in example 1.

**Table 4**

| Concentration of specific impurities in S1 at different pressures | | |
|---|---|---|
| Components | Concentration in S1 (weight-ppm) | |
| | Example 4 (3.5 bar) | Example 1 (10 bar) |
| 2-Ethyl-4-methyl-1,3-dioxolane | 0.087 | 0.052 |
| 2-Methylcyclohexanol | 0.087 | 0.052 |
| Dipropyleneglycol | 1.66 | 1.04 |
| Hydroxyacetone | 1.31 | 0.75 |
| Methylformate | 20.8 | 0.18 |
| Propylene glycol dimethyl ether (1,2-Dimethoxypropane) | 29.7 | 15.6 |

The higher concentration of side components in stream S1 led to a lower purity of the obtained products. The losses of entrainer also increased to 5 kg/h, which was almost 20% higher than in example 1. Due to the higher concentration of by-products in stream S1 and therefore in stream S2, the isolation yield and purity of the obtained products also decreased, the purity of 1-methoxypropan-2-ol in S3 decreased from 99.92 weight-% in Example 1 to 99.91 weight-% in Example 4 (0.10% less) and the isolation yield of 1-methoxypropan-2-ol also decreases from 99.85% to 99.31% (0.54% less).

The power required for the separation increased to 10 MW or 8 MW·h/t of pure methoxypropanols (1250 kg/h of methoxypropanols) as compared to only 8.9 MW or 7.12 MW·h/t of pure methoxypropanols using the inventive process.

The example demonstrated that by using the inventive method 1-methoxypropanol-2 with a purity of 99.91 weight-% could be obtained in S3 with an isolation yield of 99.31%. Additionally, 2-methoxypropanol-1 with a purity of 99.86 weight-% could be obtained as S4 with an isolation yield of 99.87%.

### Comparative Example 1

Herein, the same set-up with the same columns B to E as in Example 1 was used, the difference to Example 1 was that in column B a pressure of 1 bar was used and, consequently, in column C a pressure of also 1 bar had to be used.

Having 1 bar in column B influenced the pressure of the rest of the columns in the process. In Example 1, column B was designed for 10 bar and column C for 2 bar, creating a pressure drop helping to reduce drastically the heat demand in the second reboiler of column C. Column D was designed for 3 bars for the separation of the isomers 1-methoxylpronan-2-ol and 2-methoxypropan-1-ol, after optimizing the heat in the reboiler of column D. Using only 1 bar in column B resulted in a loss for the heat integration with column C, and also for the heat demand in column B (reboiler). This resulted in the fact that if column B was operated at 1 bar, column C also had to be operated at 1 bar. Table 5 shows the amounts of water and of specific impurities in stream S1 when column B was operated at 1 bar, compared to the amounts of water and the same specific impurities in S1 when column B was operated at 3.5 bar and at 10 bar (as in Example 1), Table 6 shows a reduced overview of all streams when column B, and consequently, all further columns C, D and E, were operated at 1 bar.

**Table 5**

| Water and specific impurities in S1 when column B was operated at different pressures | | | |
|---|---|---|---|
| | Concentration in S1 (weight-ppm, unless otherwise indicated) | | |
| Components | 1 bar | 3.5 bar | 10 bar (Example 1) |
| Water | 72.87 weight-% | 62.48 weight-% | 59.98 weight-% |
| Dipropyleneglycol | 2.52 | 1.66 | 1.04 |
| Hydroxyacetone | 3.16 | 1.31 | 0.75 |
| Methylformate | 13.5 | 20.8 | 0.18 |
| Propylene glycol dimethyl ether | 91.8 | 29.7 | 15.6 |

**Table 6**

| Parameters and content of specific compounds of streams S0 to S5, including benzene streams to column C and steam stream to column E for all columns B to E operated at 1 bar. | | | | | | | | | | | | |
|---|---|---|---|---|---|---|---|---|---|---|---|---|
| Streams | **S0** | **S1a** | **S1** | **S1** heated up (feed to column C) | **Benzene** (fresh, stream directed to column C) | **Benzene** (recycled, stream directed to column C) | **S5** | **S2** | **S2a** | **Steam** (H₂O (g), stream directed to column E) | **S3** | **S4** |
| Parameter | | | | | | | | | | | | |
| Temperature [°C] | 181 | 99.66 | 95.3 | 175 | 20 | 68.9 | 99.57 | 124.67 | 68.92 | 143 | 115.84 | 131.42 |
| Pressure [bar] | 1 | 1 | 1 | 1 | 1 | 1 | 1 | 1 | 1 | 4 | 1 | 1 |
| Mass flow [kg/h] | 11415 | 5394.805 | 6020.195 | 6020.195 | 1.13 | 48160 | 176.17 | 1621.5 | 52736 | 400 | 792.93 | 828.617 |

| Components, indic ated in wei ght-% | | | | | | | | | | | | |
|---|---|---|---|---|---|---|---|---|---|---|---|---|
| **2-Methoxypropan-1-ol** | **7.26** | **1.00E-02** | **13.87** | **13.87** | | **3.12E-07** | **4.77E-07** | **51.07** | **3.09E-07** | | **5.00E-02** | **99.89** |
| Water | 85.17 | 98.8 | 72.87 | 72.87 | | 0.29 | 91.74 | 0.01 | 8.91 | 100 | 1.99E-02 | 0.00E+00 |
| Benzene | | | | | 100 | 99.52 | 7.18 | 2.36E-09 | 90.91 | | 4.77E-09 | 0.00E+00 |
| **1-Methoxypropan-2-ol** | **6.95** | **2.46E-06** | **13.18** | **13.18** | | **0.8267 E-03** | **1.42 E-03** | **48.84** | **0.08 E-02** | | **99.86** | **3.00E-02** |

| | | | | | | | | | | | | |
|---|---|---|---|---|---|---|---|---|---|---|---|---|
| **Propylene glycol dimethyl ether (1,2-Dimethoxypropane)** | **6.50E-03** | **3.56E-03** | **9.18E-03** | **9.18E-03** | | **9.37E-04** | **4.09E-03** | **2.37E-02** | **1.18E-03** | | **4.82E-02** | **6.43E-12** |

Operation of column B at 1 bar resulted in more water going over the top of column B with stream S1, which included the isomers 1-methoxylpronan-2-ol and 2-methoxypropan-1-ol to the column C. Further, more impurities were transferred together with the water/1-methoxylpronan-2-ol, 2-methoxypropan-1-ol azeotrope to column C; S1 contained 9.18 x 10⁻³ weight-% propylene glycol dimethyl ether (more than the fivefold amount than at 10 bar as in Example 1).

Based on column B operated at only 1 bar, column C and all further columns D and E had to be also operated at 1 bar. For column C operated at 1 bar, the heat demand in the reboiler of column C increased. As outlined in Example 1, Column 2 was designed to break the azeotrope of water and 1-methoxylpronan-2-ol, 2-methoxypropan-1-ol with benzene as entrainer.

In order to have a sufficient water removal in column C, i.e. to have a water content in S2 of at the outmost 100 ppm, based on the total weight of S2, the reboiler of column C required thermal energy in an amount of 50.125 MW. Furthermore, an additional heat exchanger was required to heat up stream S1 before entering column C in order to achieve a temperature of S1 of 175 °C in accordance with S1 of Example 1 since S1, if column B was operated at only 1 bar, had only a temperature of 93.5 °C. Further, in view of the higher amount of water, which left column C via stream S2, it was necessary to add a benzene separation unit, for example, a decanter, between columns C and E. Benzene separated in said decanter from stream S2a had to be recycled to column C, otherwise, the demand on fresh benzene would have been very extensive. Furthermore, in order to separate water and benzene efficiently in column E, it was necessary to add 400 kg/h steam at 143°C and 4 bar in column E.

In view of the much higher energy demand when column B is operated at 1 bar, no heat integration was possible: operating column B at only 1 bar required an energy intake in the reboilers of columns B, C and D of 70.8 MW. Additionally, 400 kg/h of 4 bar stream (143°C) were required in column E to separate the benzene from the water.

Also the impurity content in the final stream S3 was higher when column B was operated at 1 bar: The propylene glycol dimethyl ether content in S3, based on the total weight of S3, was 0.0482 weight-% compared to only 0.00552 weight-% as in Example 1. That is, operating column B, and, consequently, also the further column C at 1 bar did not allow to have less than 0.006 weight-% of propylene glycol dimethyl ether in S3 based on the total weight of S3.

Further, even if much more energy is applied to the process, also the purity of S3 regarding 1-methoxypropan-2-ol was still a bit worse compared to Example 1: If column B was operated at 1 bar, 1-methoxypropanol-2 with a purity of only 99.86 weight-% could be obtained as S3.

### Short description of the Figures

- Fig. 1: shows the process for separating 1-methoxy-2-propanol and the involved columns B to E schematically, together with a first option of heat integration, wherein the thermal energy (heat) of the condenser of column B, i.e. of stream S1, is used to heat the reboiler of column C in that thermal energy of stream S1 is partly transferred to a heat transfer medium stream HTMS1 in a heat exchanger H. The resulting heat transfer medium stream HTMS1a, which has an increased thermal energy content compared to HTMS1, is used to provide thermal energy to a heat exchanger unit, which supplies the reboiler of column C.
- Fig. 2: shows the process for separating 1-methoxy-2-propanol and the involved columns B to E schematically, together with a second option of heat integration, wherein the thermal energy (heat) of the condenser of column B, i.e. of stream S1, is used to heat the reboiler of column D in that thermal energy of stream S1 is partly transferred to a heat transfer medium stream HTMS1 in a heat exchanger H. The resulting heat transfer medium stream HTMS1a, which has an increased thermal energy content compared to HTMS1, is used to provide thermal energy to a heat exchanger unit, which supplies the reboiler of column D.
- Fig. 3: shows the process for separating 1-methoxy-2-propanol and the involved columns B to E schematically, together with a third option of heat integration, wherein the thermal energy (heat) of the condenser of column B, i.e. of stream S1, is used to heat the reboiler of columns C and D in that thermal energy of stream S1 is partly transferred to a heat transfer medium stream HTMS1 in a heat exchanger H. The resulting heat transfer medium stream HTMS1a, which has an increased thermal energy content compared to HTMS1, is used to provide thermal energy to a heat exchanger unit, which supplies the reboiler of column C and to a heat exchanger unit, which supplies the reboiler of column D.
- Fig. 4: shows the process for separating 1-methoxy-2-propanol and the involved columns B to E schematically, together with a fourth option of heat integration, wherein the thermal energy of stream S1a is used to produce steam (gaseous H₂O), which is used in column E for the steam stripping. Thermal energy of stream S1a is partly transferred to a heat transfer medium stream HTMS2, wherein a heat transfer medium stream HTMS2a is obtained which has an increased thermal energy content compared to HTMS2. HTMS2a is then used to produce the steam required for column E. In addition, the thermal energy (heat) of the condenser of column B, i.e. of stream S1, is used to heat the reboiler of column C in that thermal energy of stream S1 is partly transferred to a heat transfer medium stream HTMS1 in a heat exchanger H. The resulting heat transfer medium stream HTMS1a, which has an increased thermal energy content compared to HTMS1, is used to provide thermal energy to a heat exchanger unit, which supplies the reboiler of column C.
- Fig. 5: shows the process for separating 1-methoxy-2-propanol and the involved columns B to E schematically, together with a fifth, complete, heat integration. The thermal energy (heat) of the condenser of column B, i.e. of stream S1, is used to heat the reboiler of columns C and D in that thermal energy of stream S1 is partly transferred to a heat transfer medium stream HTMS1 in a heat exchanger H, wherein a heat transfer medium stream HTMS1a is obtained which has an increased thermal energy content compared to HTMS1. The heat transfer medium stream HTMS1a is used to provide thermal energy to a heat exchanger unit, which supplies the reboiler of column C and to heat exchanger unit, which supplies the reboiler of column D. In addition, the thermal energy of stream S1a is used to produce the steam needed in column E in that thermal energy of stream S1a is partly transferred to a heat transfer medium stream HTMS2 in a heat exchanger H1, wherein a heat transfer medium stream HTMS2a is obtained which has an increased thermal energy content compared to HTMS2. HTMS2a is then used to produce the steam required for column E.

### Cited Literature

- US 5,723,024 A
- EP 1 375 462 A1
- EP 0 425 893 A
- DE 10233388 A1
- US 2004/0000473 A1
- CN 103342631
- CN 103992214

## Claims

1. A process for separating 1-methoxypropan-2-ol from an aqueous stream comprising 1-methoxypropan-2-ol and 2-methoxypropan-1-ol, wherein the process comprises:
(a) Providing a stream S0 comprising 1-methoxypropan-2-ol, 2-methoxypropan-1-ol and water, and having a molar ratio of 1-methoxypropan-2-ol : 2-methoxypropan-1-ol in the range of from 1 : 5 to 5 : 1;
(b) separating 1-methoxypropan-2-ol and 2-methoxypropan-1-ol from the stream S0 provided in (a) by distillation comprising subjecting the stream S0 provided in (a) to distillation conditions in a distillation unit comprising a distillation column B, obtaining a (top) stream S1 comprising 1-methoxypropan-2-ol, 2-methoxypropan-1-ol and water, which is enriched in 1-methoxypropan-2-ol and 2-methoxypropan-1-ol compared to the stream S0 and a bottoms stream S1a comprising water and being depleted of 1-methoxypropan-2-ol and 2-methoxypropan-1-ol compared to S0; wherein the distillation column B is operated at a pressure of ≥ 2 bar;
(c) azeotropic distillation of the stream S1 obtained in (b) with an entrainer, comprising subjecting the stream S1 to azeotropic distillation conditions in an azeotropic distillation unit comprising a distillation column C, obtaining a bottoms stream S2 which is depleted of water and further enriched in 1-methoxypropan-2-ol and 2-methoxypropan-1-ol compared to the stream S1 and a top stream S2a comprising water and the entrainer;
(d) separating 1-methoxypropan-2-ol from the stream S2 obtained in (c) by distillation, comprising subjecting the stream S2 obtained in (c) to distillation conditions in a distillation unit comprising a distillation column D, obtaining a stream S3 comprising ≥ 95 weight-% 1-methoxypropan-2-ol and ≤ 0.5 weight-% of 2-methoxypropan-1-ol, based on the total weight of stream S3, and a stream S4 comprising ≥ 95 weight-% 2-methoxypropan-1-ol based on the total weight of stream S4;
(e) optionally distillative separation of the stream S2a comprising water and the entrainer obtained in (c) in a steam stripping column E using steam as stripping agent, obtaining a stream S5 comprising the entrainer, which is depleted of water compared to the stream S2a, and an aqueous stream S6 being depleted of the entrainer compared to S2a; and optionally recirculating at least a part of the stream S5 to (c).

2. The process for separating 1-methoxypropan-2-ol from an aqueous stream comprising 1-methoxypropan-2-ol and 2-methoxypropan-1-ol according to claim 1, wherein the thermal energy of stream S1 is partly transferred to a heat transfer medium stream HTMS1 after (b) and before step (c), preferably in a heat exchanger H, obtaining a heat transfer medium stream HTMS1a having an increased thermal energy content compared to HTMS1; wherein HTMS1a is used to provide thermal energy to:
- the azeotropic distillation unit of step (c), preferably to a heat exchanger unit connected to the distillation column of the azeotropic distillation unit of (c),
and/or
- the distillation unit of (d), preferably to a heat exchanger unit connected to the distillation column of the distillation unit of (d).

3. The process for separating 1-methoxypropan-2-ol from an aqueous stream comprising 1-methoxypropan-2-ol and 2-methoxypropan-1-ol according to claim 2, wherein the thermal energy of the bottoms stream S1a of (b) is partly transferred to a heat transfer medium stream HTMS2, preferably in a heat exchanger H1, obtaining a heat transfer medium stream HTMS2a, which has an increased thermal energy content compared to HTMS2, wherein HTMS2a is used to provide thermal energy to the steam used as stripping agent in the steam stripping column of (e).

4. The process for separating 1-methoxypropan-2-ol from an aqueous stream comprising 1-methoxypropan-2-ol and 2-methoxypropan-1-ol according to claim 2 or 3, wherein the thermal energy provided by HTMS1a provides at least 90 % of the energy demand of the azeotropic distillation unit of (c), and/or of the distillation unit of (d), preferably the thermal energy provided by HTMS1a provides at least 95 % of the energy demand of the azeotropic distillation unit of (c), and/or of the distillation unit of (d); more preferred the thermal energy provided by HTMS1a provides at least 98 % of the energy demand of the azeotropic distillation unit of (c), and/or of the distillation unit of (d).

5. The process for separating 1-methoxypropan-2-ol from an aqueous stream comprising 1-methoxypropan-2-ol and 2-methoxypropan-1-ol according to any one of claims 1 to 4, wherein stream S0 provided in (a) comprises water in an amount in the range of from 50 to 90 weight-%, preferably in the range of from 55 to 85 weight-% and a mixture of 1-methoxypropan-2-ol and 2-methoxypropan-1-ol in an amount in the range of from 8 to 50 weight-%, preferably in the range of from 13 to 45 weight-%, each based on the total weight of stream S0, the remaining amount up to 100 weight-% being other components (impurities and solvent (MeOH)).

6. The process for separating 1-methoxypropan-2-ol from an aqueous stream comprising 1-methoxypropan-2-ol and 2-methoxypropan-1-ol according to any one of claims 1 to 5, wherein stream S0 provided in (a) comprises 1-methoxypropan-2-ol and 2-methoxypropan-1-ol in a molar ratio in the range of from 1 : 4 to 4 : 1; preferably in the range of from 1 : 3 to 3 : 1, more preferred in the range of from 1 : 2 to 2 : 1, more preferred in the range of from 0.75 : 1 to 1 : 0.75.

7. The process for separating 1-methoxypropan-2-ol from an aqueous stream comprising 1-methoxypropan-2-ol and 2-methoxypropan-1-ol according to any one of claims 1 to 6, wherein stream S0 provided in (a) comprises propylene glycol dimethyl ether in an amount of ≥ 0.001 weight-%, more preferred ≥ 0.006 weight-%, more preferred in the range of from 0.001 to 0.1 weight-%, preferably in the range of from 0.003 to 0.01 weight-%, more preferred in the range of from 0.004 to 0.01 weight-%, more preferred in the range of from 0.005 to 0.008 weight-%, based on the total weight of S0.

8. The process for separating 1-methoxypropan-2-ol from an aqueous stream comprising 1-methoxypropan-2-ol and 2-methoxypropan-1-ol according to any one of claims 1 to 7, wherein the distillation column B comprised in the distillation unit according to (b) is operated at a pressure in the range of from 2 to 30 bar, preferably in the range of from 3.5 to 20 bar, more preferred in the range of from 4 to 20 bar, more preferred in the range of from >5 to 15 bar, more preferred in the range of from 5.5 to 15 bar, more preferred in the range of from 6 to 14 bar, more preferred in the range of from 7 to 12 bar.

9. The process for separating 1-methoxypropan-2-ol from an aqueous stream comprising 1-methoxypropan-2-ol and 2-methoxypropan-1-ol according to any one of claims 1 to 8, wherein ≥ 95 weight-% of stream S1, which leaves distillation column B over the top, consist of water,1-methoxypropan-2-ol and 2-methoxypropan-1-ol; wherein preferably stream S1 comprises water in a amount in the range of from 40 to 80 weight-%, preferably in the range of from 50 to 70 weight-%, more preferred in the range of from 55 to 65 weight-%; and a mixture of 1-methoxypropan-2-ol and 2-methoxypropan-1-ol in an amount in the range of from 20 to 60 weight-%, preferably in the range of from 30 to 50 weight-%, more preferred in the range of from 35 to 45 weight-%, each based on the total weight of stream S1, wherein preferably stream S1 comprises 1-methoxypropan-2-ol and 2-methoxypropan-1-ol in a molar ratio in the range of from 1 : 4 to 4 : 1; preferably in the range of from 1 : 3 to 3 : 1, more preferred in the range of from 1 : 2 to 2 : 1, more preferred in the range of from 0.75 : 1 to 1 : 0.75.

10. The process for separating 1-methoxypropan-2-ol from an aqueous stream comprising 1-methoxypropan-2-ol and 2-methoxypropan-1-ol according to any one of claims 1 to 9, wherein stream S1, which leaves distillation column B over the top, contains less than 0.05 weight-%, preferably less than 0.005 weight-%, more preferred less than 0.004 weight-%, more preferred less than 0.002 weight-%, of propylene glycol dimethyl ether, based on the total weight of S1.

11. The process for separating 1-methoxypropan-2-ol from an aqueous stream comprising 1-methoxypropan-2-ol and 2-methoxypropan-1-ol according to any one of claims 1 to 10, wherein the stream S3 is removed as top stream from distillation column D, which comprises ≥ 95 weight-% 1-methoxypropan-2-ol and ≤ 0.5 weight-% of 2-methoxypropan-1-ol, preferably ≥ 98 weight-% 1-methoxypropan-2-ol and ≤ 0.15 weight-% of 2-methoxypropan-1-ol, more preferred ≥ 99 weight-% 1-methoxypropan-2-ol and ≤ 0.1 weight-% of 2-methoxypropan-1-ol, each based on the total weight of stream S3; and/or wherein the stream S3 comprises less than 0.01 weight-%, preferably less than 0.008 weight-%, more preferred less than 0.007 weight-%, more preferred less than 0.006 weight-%, of propylene glycol dimethyl ether based on the total weight of stream S3.

## Patentansprüche

1. Verfahren zum Abtrennen von 1-Methoxypropan-2-ol aus einem wässrigen Strom umfassend 1-Methoxypropan-2-ol und 2-Methoxypropan-1-ol, wobei das Verfahren umfasst:
(a) Bereitstellen eines Stroms S0 umfassend 1-Methoxypropan-2-ol, 2-Methoxypropan-1-ol und Wasser und mit einem Molverhältnis von 1-Methoxypropan-2-ol : 2-Methoxypropan-1-ol im Bereich von 1 : 5 bis 5 : 1;
(b) Abtrennen von 1-Methoxypropan-2-ol und 2-Methoxypropan-1-ol aus dem in (a) bereitgestellten Strom S0 durch Destillation, umfassend das Aussetzen des in (a) bereitgestellten Stroms S0 Destillationsbedingungen in einer Destillationseinheit umfassend eine Destillationskolonne B, um einen (Kopf-) Strom S1 umfassend 1-Methoxypropan-2-ol, 2-Methoxypropan-1-ol und Wasser, der im Vergleich zu dem Strom S0 mit 1-Methoxypropan-2-ol und 2-Methoxypropan-1-ol angereichert ist, und einen Sumpfstrom S1a umfassend Wasser, der im Vergleich zu S0 von 1-Methoxypropan-2-ol und 2-Methoxypropan-1-ol abgereichert ist, zu erhalten; wobei die Destillationskolonne B bei einem Druck von ≥ 2 bar betrieben wird;
(c) azeotrope Destillation des in (b) erhaltenen Stroms S1 mit einem Schleppmittel, umfassend das Aussetzen des Stroms S1 azeotropen Destillationsbedingungen in einer azeotropen Destillationseinheit umfassend eine Destillationssäule C, um einen Sumpfstrom S2, der von Wasser abgereichert und ferner im Vergleich zu Strom S1 mit 1-Methoxypropan-2-ol und 2-Methoxypropan-1-ol angereichert ist, und einen Kopfstrom S2a umfassend Wasser und das Schleppmittel zu erhalten;
(d) Abtrennen von 1-Methoxypropan-2-ol von dem in (c) erhaltenen Strom S2 durch Destillation, umfassend Aussetzen des in (c) erhaltenen Stroms S2 Destillationsbedingungen in einer Destillationseinheit umfassend eine Destillationskolonne D, um einen Strom S3 umfassend ≥ 95 Gew.-% 1-Methoxypropan-2-ol und ≤ 0,5 Gew.-% 2-Methoxypropan-1-ol, bezogen auf das Gesamtgewicht von Strom S3, und einen Strom S4 umfassend ≥ 95 Gew.-% 2-Methoxypropan-1-ol, bezogen auf das Gesamtgewicht von Strom S4, zu erhalten;
(e) gegebenenfalls destillative Abtrennung des in (c) erhaltenen Stroms S2a, der Wasser und das Schleppmittel umfasst, in einer Dampfstrippkolonne E unter Verwendung von Dampf als Strippmittel, Erhalten eines Stroms S5, der das Schleppmittel umfasst, das im Vergleich zu dem Strom S2a von Wasser abgereichert ist, und eines wässrigen Stroms S6, der im Vergleich zu S2a von dem Schleppmittel abgereichert ist; und gegebenenfalls Rezirkulieren zumindest eines Teils des Stroms S5 in (c).

2. Verfahren zum Abtrennen von 1-Methoxypropan-2-ol aus einem wässrigen Strom umfassend 1-Methoxypropan-2-ol und 2-Methoxypropan-1-ol nach Anspruch 1, wobei die Wärmeenergie von Strom S1 nach (b) und vor Schritt (c) teilweise auf einen Wärmeträgermediumstrom HTMS1 übertragen wird, bevorzugt in einem Wärmetauscher H, um einen Wärmeträgermediumstrom HTMS1a mit einem im Vergleich zu HTMS1 erhöhten Wärmeenergieinhalt zu erhalten; wobei HTMS1a verwendet wird, um Wärmeenergie bereitzustellen für:
- die azeotrope Destillationseinheit von Schritt (c), bevorzugt an eine Wärmetauschereinheit, die mit der Destillationskolonne der azeotropen Destillationseinheit von (c) verbunden ist,
und/oder
- die Destillationseinheit von (d), bevorzugt an eine Wärmetauschereinheit, die mit der Destillationskolonne der Destillationseinheit von (d) verbunden ist.

3. Verfahren zum Abtrennen von 1-Methoxypropan-2-ol aus einem wässrigen Strom umfassend 1-Methoxypropan-2-ol und 2-Methoxypropan-1-ol nach Anspruch 2, wobei die Wärmeenergie des Sumpfstroms S1a aus (b) teilweise an einen Wärmeübertragungsmediumstrom HTMS2 übertragen wird, bevorzugt in einem Wärmetauscher H1, um einen Wärmeübertragungsmediumstrom HTMS2a zu erhalten, der im Vergleich zu HTMS2 einen erhöhten Wärmeenergiegehalt aufweist, wobei HTMS2a verwendet wird, um dem als Strippmittel in der Dampfstrippkolonne von (e) verwendeten Dampf Wärmeenergie bereitzustellen.

4. Verfahren zum Abtrennen von 1-Methoxypropan-2-ol aus einem wässrigen Strom umfassend 1-Methoxypropan-2-ol und 2-Methoxypropan-1-ol nach Anspruch 2 oder 3, wobei die von HTMS1a bereitgestellte Wärmeenergie wenigstens 90 % des Energiebedarfs der azeotropen Destillationseinheit von (c) und/oder der Destillationseinheit von (d) bereitstellt, bevorzugt wobei die von HTMS1a bereitgestellte Wärmeenergie wenigstens 95 % des Energiebedarfs der azeotropen Destillationseinheit von (c) und/oder der Destillationseinheit von (d) bereitstellt; mehr bevorzugt wobei die von HTMS1a bereitgestellte Wärmeenergie wenigstens 98 %, des Energiebedarfs der azeotropen Destillationseinheit von (c) und/oder der Destillationseinheit von (d) bereitstellt.

5. Verfahren zum Abtrennen von 1-Methoxypropan-2-ol aus einem wässrigen Strom umfassend 1-Methoxypropan-2-ol und 2-Methoxypropan-1-ol nach einem der Ansprüche 1 bis 4, wobei der in (a) bereitgestellte Strom S0 Wasser in einer Menge im Bereich von 50 bis 90 Gew.-%, bevorzugt im Bereich von 55 bis 85 Gew.-%, und ein Gemisch von 1-Methoxypropan-2-ol und 2-Methoxypropan-1-ol in einer Menge im Bereich von 8 bis 50 Gew.-%, bevorzugt im Bereich von 13 bis 45 Gew.-%, umfasst, jeweils bezogen auf das Gesamtgewicht von Strom S0, wobei die verbleibende Menge auf 100 Gew.-% andere Komponenten (Verunreinigungen und Lösungsmittel (MeOH)) sind.

6. Verfahren zum Abtrennen von 1-Methoxypropan-2-ol aus einem wässrigen Strom umfassend 1-Methoxypropan-2-ol und 2-Methoxypropan-1-ol nach einem der Ansprüche 1 bis 5, wobei der in (a) bereitgestellte Strom S0 1-Methoxypropan-2-ol und 2-Methoxypropan-1-ol in einem Molverhältnis im Bereich von 1 : 4 bis 4 : 1; bevorzugt im Bereich von 1 : 3 bis 3 : 1, mehr bevorzugt im Bereich von 1 : 2 bis 2 : 1, mehr bevorzugt im Bereich von 0,75 : 1 bis 1 : 0,75.

7. Verfahren zum Abtrennen von 1-Methoxypropan-2-ol aus einem wässrigen Strom umfassend 1-Methoxypropan-2-ol und 2-Methoxypropan-1-ol nach einem der Ansprüche 1 bis 6, wobei der in (a) bereitgestellte Strom S0 Propylenglycoldimethylether in einer Menge von ≥ 0,001 Gew.-%, mehr bevorzugt ≥ 0,006 Gew.-%, mehr bevorzugt im Bereich von 0,001 bis 0,1 Gew.-%, bevorzugt im Bereich von 0,003 bis 0,01 Gew.-%, mehr bevorzugt im Bereich von 0,004 bis 0,01 Gew.-%, mehr bevorzugt im Bereich von 0,005 bis 0,008 Gew.-%, bezogen auf das Gesamtgewicht von S0, umfasst.

8. Verfahren zum Abtrennen von 1-Methoxypropan-2-ol aus einem wässrigen Strom umfassend 1-Methoxypropan-2-ol und 2-Methoxypropan-1-ol nach einem der Ansprüche 1 bis 7, wobei die von der Destillationseinheit gemäß (b) umfasste Destillationskolonne B bei einem Druck im Bereich von 2 bis 30 bar, bevorzugt im Bereich von 3,5 bis 20 bar, mehr bevorzugt im Bereich von 4 bis 20 bar, mehr bevorzugt im Bereich von > 5 bis 15 bar, mehr bevorzugt im Bereich von 5,5 bis 15 bar, mehr bevorzugt im Bereich von 6 bis 14 bar, mehr bevorzugt im Bereich von 7 bis 12 bar, betrieben wird.

9. Verfahren zum Abtrennen von 1-Methoxypropan-2-ol aus einem wässrigen Strom umfassend 1-Methoxypropan-2-ol und 2-Methoxypropan-1-ol nach einem der Ansprüche 1 bis 8, wobei ≥ 95 Gew.-% des Stroms S1, der die Destillationssäule B über den Kopf verlässt, aus Wasser, 1-Methoxypropan-2-ol und 2-Methoxypropan-1-ol besteht; wobei bevorzugt Strom S1 Wasser in einer Menge im Bereich von 40 bis 80 Gew.-%, bevorzugt im Bereich von 50 bis 70 Gew.-%, mehr bevorzugt im Bereich von 55 bis 65 Gew.-% umfasst; und ein Gemisch von 1-Methoxypropan-2-ol und 2-Methoxypropan-1-ol in einer Menge im Bereich von 20 bis 60 Gew.-%, bevorzugt im Bereich von 30 bis 50 Gew.-%, mehr bevorzugt im Bereich von 35 bis 45 Gew.-%, jeweils bezogen auf das Gesamtgewicht von Strom S1, wobei bevorzugt Strom S1 1-Methoxypropan-2-ol und 2-Methoxypro-pan-1-ol in einem Molverhältnis im Bereich von 1 : 4 bis 4 : 1; bevorzugt im Bereich von 1 : 3 bis 3 : 1, mehr bevorzugt im Bereich von 1 : 2 bis 2 : 1, mehr bevorzugt im Bereich von 0,75 : 1 bis 1 : 0,75.

10. Verfahren zum Abtrennen von 1-Methoxypropan-2-ol aus einem wässrigen Strom umfassend 1-Methoxypropan-2-ol und 2-Methoxypropan-1-ol nach einem der Ansprüche 1 bis 9, wobei der Strom S1, der aus der Destillationskolonne B über den Kopf austritt, weniger als 0,05 Gew.-%, bevorzugt weniger als 0,005 Gew.-%, mehr bevorzugt weniger als 0,004 Gew.-%, mehr bevorzugt weniger als 0,002 Gew.-%, Propylenglycoldimethylether, bezogen auf das Gesamtgewicht von S1, enthält.

11. Verfahren zum Abtrennen von 1-Methoxypropan-2-ol aus einem wässrigen Strom umfassend 1-Methoxypropan-2-ol und 2-Methoxypropan-1-ol nach einem der Ansprüche 1 bis 10, wobei der Strom S3 als Kopfstrom aus der Destillationskolonne D entnommen wird, der ≥ 95 Gew.-% 1-Methoxypropan-2-ol und ≤ 0,5 Gew.-% 2-Methoxypropan-1-ol, bevorzugt ≥ 98 Gew.-% 1-Methoxypropan-2-ol und ≤ 0,15 Gew.-% 2-Methoxypropan-1-ol, mehr bevorzugt ≥ 99 Gew.-% 1-Methoxypropan-2-ol und ≤ 0,1 Gew.-% 2-Methoxypropan-1-ol, jeweils bezogen auf das Gesamtgewicht von Strom S3, umfasst; und/oder wobei der Strom S3 weniger als 0,01 Gew.-%, bevorzugt weniger als 0,008 Gew.-%, mehr bevorzugt weniger als 0,007 Gew.-%, mehr bevorzugt weniger als 0,006 Gew.-%, Propylenglycoldimethylether, bezogen auf das Gesamtgewicht von Strom S3, umfasst.

## Revendications

1. Procédé de séparation de 1-méthoxypropan-2-ol d'un flux aqueux comprenant du 1-méthoxypropan-2-ol et du 2-méthoxypropan-1-ol, le procédé comprenant :
(a) la fourniture d'un flux S0 comprenant du 1-méthoxypropan-2-ol, du 2-méthoxypropan-1-ol et de l'eau, et ayant un rapport molaire de 1-méthoxypropan-2-ol : 2-méthoxypropan-1-ol dans la plage de 1 : 5 à 5 : 1 ;
(b) la séparation du 1-méthoxypropan-2-ol et du 2-méthoxypropan-1-ol du flux S0 fourni en (a) par distillation comprenant la soumission du flux S0 fourni en (a) à des conditions de distillation dans une unité de distillation comprenant une colonne de distillation B, de façon à obtenir un flux (de tête) S1 comprenant du 1-méthoxypropan-2-ol, du 2-méthoxypropan-1-ol et de l'eau, qui est enrichi en 1-méthoxypropan-2-ol et en 2-méthoxypropan-1-ol par rapport au flux S0 et un flux de fond S1a comprenant de l'eau et étant appauvri en 1-méthoxypropan-2-ol et en 2-méthoxypropan-1-ol par rapport à S0 ; la colonne de distillation B fonctionnant à une pression ≥ 2 bar ;
(c) la distillation azéotropique du flux S1 obtenu en
(b) avec un agent d'entraînement, comprenant la soumission du flux S1 à des conditions de distillation azéotropique dans une unité de distillation azéotropique comprenant une colonne de distillation C, de façon à obtenir un flux de fond S2 appauvri en eau et en outre enrichi en 1-méthoxypropan-2-ol et en 2-méthoxypro-pan-1-ol par rapport au flux S1 et un flux de tête S2a comprenant de l'eau et l'agent d'entraînement ;
(d) la séparation du 1-méthoxypropan-2-ol du flux S2 obtenu en (c) par distillation, comprenant la soumission du flux S2 obtenu en (c) à des conditions de distillation dans une unité de distillation comprenant une colonne de distillation D, de façon à obtenir un flux S3 comprenant ≥ 95 % en poids de 1-méthoxypropan-2-ol et ≤ 0,5 % en poids de 2-méthoxypropan-1-ol, par rapport au poids total du flux S3, et un flux S4 comprenant ≥ 95 % en poids de 2-méthoxypropan-1-ol par rapport au poids total du flux S4 ;
(e) éventuellement la séparation par distillation du flux S2a comprenant de l'eau et l'agent d'entraînement obtenu en (c) dans une colonne E de strippage à la vapeur en utilisant de la vapeur comme agent de strippage, de façon à obtenir un flux S5 comprenant l'agent d'entraînement appauvri en eau par rapport au flux S2a, et un flux aqueux S6 appauvri en agent d'entraînement par rapport à S2a ; et éventuellement la recirculation d'au moins une partie du flux S5 vers (c).

2. Procédé de séparation de 1-méthoxypropan-2-ol d'un flux aqueux comprenant du 1-méthoxypropan-2-ol et du 2-méthoxypropan-1-ol selon la revendication 1, dans lequel l'énergie thermique du flux S1 est partiellement transférée à un flux de fluide caloporteur HTMS1 après (b) et avant l'étape (c), de préférence dans un échangeur de chaleur H, de façon à obtenir un flux de fluide caloporteur HTMS1a ayant une teneur en énergie thermique accrue par rapport à HTMS1 ; HTMS1a étant utilisé pour fournir de l'énergie thermique à :
- l'unité de distillation azéotropique de l'étape (c), de préférence à une unité d'échangeur de chaleur reliée à la colonne de distillation de l'unité de distillation azéotropique de l'étape (c),
et/ou
- l'unité de distillation de (d), de préférence à une unité d'échangeur de chaleur reliée à la colonne de distillation de l'unité de distillation de (d).

3. Procédé de séparation de 1-méthoxypropan-2-ol d'un flux aqueux comprenant du 1-méthoxypropan-2-ol et du 2-méthoxypropan-1-ol selon la revendication 2, dans lequel l'énergie thermique du flux de fond S1a de (b) est partiellement transférée à un flux de fluide caloporteur HTMS2, de préférence dans un échangeur de chaleur H1, de façon à obtenir un flux de fluide caloporteur HTMS2a, qui a une teneur en énergie thermique accrue par rapport à HTMS2, dans lequel HTMS2a est utilisé pour fournir de l'énergie thermique à la vapeur utilisée comme agent de strippage dans la colonne de strippage à la vapeur de (e) .

4. Procédé de séparation de 1-méthoxypropan-2-ol d'un flux aqueux comprenant du 1-méthoxypropan-2-ol et du 2-méthoxypropan-1-ol selon la revendication 2 ou 3, dans lequel l'énergie thermique fournie par HTMS1a fournit au moins 90 % de la demande énergétique de l'unité de distillation azéotropique de (c), et/ou de l'unité de distillation de (d), de préférence l'énergie thermique fournie par HTMS1a fournit au moins 95 % de la demande énergétique de l'unité de distillation azéotropique de (c), et/ou de l'unité de distillation de (d) ; plus préférablement, l'énergie thermique fournie par HTMS1a fournit au moins 98 % de la demande énergétique de l'unité de distillation azéotropique de (c), et/ou de l'unité de distillation de (d).

5. Procédé de séparation de 1-méthoxypropan-2-ol d'un flux aqueux comprenant du 1-méthoxypropan-2-ol et du 2-méthoxypropan-1-ol selon l'une quelconque des revendications 1 à 4, dans lequel le flux S0 fourni en (a) comprend de l'eau en une quantité dans la plage de 50 à 90 % en poids, de préférence dans la plage de 55 à 85 % en poids et un mélange de 1-méthoxypropan-2-ol et de 2-méthoxypropan-1-ol en une quantité dans la plage de 8 à 50 % en poids, de préférence dans la plage de 13 à 45 % en poids, chacun par rapport au poids total du flux S0, le complément à 100 % en poids étant constitué d'autres composants (impuretés et solvant (MeOH)).

6. Procédé de séparation de 1-méthoxypropan-2-ol d'un flux aqueux comprenant du 1-méthoxypropan-2-ol et du 2-méthoxypropan-1-ol selon l'une quelconque des revendications 1 à 5, dans lequel le flux S0 fourni en (a) comprend du 1-méthoxypropan-2-ol et du 2-méthoxypropan-1-ol en un rapport molaire dans la plage allant de 1 : 4 à 4 : 1 ; de préférence dans la plage allant de 1 : 3 à 3 : 1, plus préférablement dans la plage allant de 1 : 2 à 2 : 1, plus préférablement dans la plage allant de 0,75 : 1 à 1 : 0,75.

7. Procédé de séparation de 1-méthoxypropan-2-ol d'un flux aqueux comprenant du 1-méthoxypropan-2-ol et du 2-méthoxypropan-1-ol selon l'une quelconque des revendications 1 à 6, dans lequel le flux S0 fourni en (a) comprend de l'éther diméthylique de propylène glycol en une quantité de ≥ 0,001 % en poids, plus préférablement ≥ 0,006 % en poids, plus préférablement dans la plage de 0,001 à 0,1 % en poids, plus préférablement dans la plage de 0,003 à 0,01 % en poids, plus préférablement dans la plage de 0,004 à 0,01 % en poids, plus préférablement dans la plage de 0,005 à 0,008 % en poids, par rapport au poids total de S0.

8. Procédé de séparation de 1-méthoxypropan-2-ol d'un flux aqueux comprenant du 1-méthoxypropan-2-ol et du 2-méthoxypropan-1-ol selon l'une quelconque des revendications 1 à 7, dans lequel la colonne de distillation B comprise dans l'unité de distillation selon (b) fonctionne à une pression dans la plage de 2 à 30 bars, préférablement dans la plage allant de 3,5 à 20 bars, plus préférablement dans la plage de 4 à 20 bars, plus préférablement dans la plage de > 5 à 15 bars, plus préférablement dans la plage de 5,5 à 15 bars, plus préférablement dans la plage de 6 à 14 bars, plus préférablement dans la plage de 7 à 12 bars.

9. Procédé de séparation de 1-méthoxypropan-2-ol d'un flux aqueux comprenant du 1-méthoxypropan-2-ol et du 2-méthoxypropan-1-ol selon l'une quelconque des revendications 1 à 8, dans lequel ≥ 95% en poids du flux S1, qui sort de la colonne de distillation B par le haut, sont constitués d'eau, de 1-méthoxypropan-2-ol et de 2-méthoxypropan-1-ol ; dans lequel, de préférence, le flux S1 comprend de l'eau en une quantité dans la plage allant de 40 à 80 % en poids, de préférence dans la plage allant de 50 à 70 % en poids, plus préférablement dans la plage allant de 55 à 65 % en poids ; et un mélange de 1-méthoxypropan-2-ol et de 2-méthoxypropan-1-ol en une quantité dans la plage allant de 20 à 60 % en poids, de préférence dans la plage allant de 30 à 50 % en poids, plus préférablement dans la plage allant de 35 à 45 % en poids, chacun sur la base du poids total du flux S1, le flux S1 comprenant de préférence du 1-méthoxypropan-2-ol et du 2-méthoxypro-pan-1-ol en un rapport molaire compris dans la plage allant de 1 : 4 à 4 : 1 ; de préférence dans la plage allant de 1 : 3 à 3 : 1, plus préférablement dans la plage allant de 1 : 2 à 2 : 1, plus préférablement dans la plage allant de 0,75 : 1 à 1 : 0,75.

10. Procédé de séparation de 1-méthoxypropan-2-ol d'un flux aqueux comprenant du 1-méthoxypropan-2-ol et du 2-méthoxypropan-1-ol selon l'une quelconque des revendications 1 à 9, dans lequel le flux S1, qui sort de la colonne de distillation B par le haut, contient moins de 0,05 % en poids, de préférence moins de 0,005 % en poids, plus préférablement moins de 0,004 % en poids, plus préférablement moins de 0,002 % en poids, d'éther diméthylique de propylène glycol, par rapport au poids total de S1.

11. Procédé de séparation de 1-méthoxypropan-2-ol d'un flux aqueux comprenant du 1-méthoxypropan-2-ol et du 2-méthoxypropan-1-ol selon l'une quelconque des revendications 1 à 10, dans lequel le flux S3 est éliminé en tant que flux de tête de la colonne de distillation D, qui comprend ≥ 95 % en poids de 1-méthoxypropan-2-ol et ≤ 0,5 % en poids de 2-méthoxypropan-1-ol, de préférence ≥ 98 % en poids de 1-méthoxypropan-2-ol et ≤ 0,15 % en poids de 2-méthoxypropan-1-ol, plus préférablement ≥ 99 % en poids de 1-méthoxypropan-2-ol et ≤ 0,1 % en poids de 2-méthoxypropan-1-ol, chacun sur la base du poids total du flux S3 ; et/ou dans lequel le flux S3 comprend moins de 0,01 % en poids, de préférence moins de 0,008 % en poids, plus préférablement moins de 0,007 % en poids, plus préférablement moins de 0,006 % en poids, d'éther diméthylique de propylène glycol sur la base du poids total du flux S3.
